# EUROPEAN PATENT SPECIFICATION

(11) **EP 2 416 806 B1**
(45) Date of publication and mention of the grant of the patent: **14.12.2016**
(21) Application number: 09786466.4
(22) Date of filing: 10.04.2009
(51) Int. Cl.: A61K 49/00, A61K 49/04, A61K 49/08, A61K 49/22, A61K 51/04, A61K 51/00, A61K 49/12, A61K 49/18, A61K 51/06, B82Y 5/00, A61K 103/10, G01N 33/60

(54) **FUCOIDANS AS LIGANDS FOR THE DIAGNOSIS OF DEGENERATIVE PATHOLOGIES**
FUCOIDANE ALS LIGANDEN ZUR DIAGNOSE VON DEGENERATIVEN PATHOLOGIEN
FUCOÏDANES EN TANT QUE LIGANDS POUR LE DIAGNOSTIC DE PATHOLOGIES DÉGÉNÉRATIVES

(43) Date of publication of application: 15.02.2012
(60) Divisional application: 16191071.6
(73) Proprietor: Université Paris 13, 93430 Villetaneuse (FR)
(72) Inventor: MICHEL, Jean-Baptiste, F-75877 Paris Cedex 18 (FR); LETOURNEUR, Didier, F-75877 Paris Cedex 18 (FR); CHAUBET, Frédéric, F-75877 Paris Cedex 18 (FR); BACHELET, Laure, F-75877 Paris Cedex 18 (FR); ROUZET, François, F-75877 Paris Cedex 18 (FR); MEULEMANS, Alain, F-75877 Paris Cedex 18 (FR)
(74) Representative: Cabinet Plasseraud
(86) International application number: PCT/IB2009/052791
(87) International publication number: WO 2010/116209

(56) References cited:
- HARIRI G ET AL: "Radiation-Guided P-Selectin Antibody Targeted to Lung Cancer" ANNALS OF BIOMEDICAL ENGINEERING, KLUWER ACADEMIC PUBLISHERS-PLENUM PUBLISHERS, NE, vol. 36, no. 5, 14 February 2008 (2008-02-14), pages 821-830, XP019598332 ISSN: 1573-9686
- GRATZ S ET AL: "99MTC-E-SELECTIN BINDING PEPTIDE FOR IMAGING ACUTE OSTEOMYELITIS IN A NOVEL RAT MODEL" NUCLEAR MEDICINE COMMUNICATIONS, LIPPINCOTT WILLIAMS AND WILKINS, XX, vol. 22, no. 9, 1 September 2001 (2001-09-01), pages 1003-1013, XP009076103 ISSN: 0143-3636
- BACHELET L ET AL: "Affinity of low molecular weight fucoidan for P-selectin triggers its binding to activated human platelets" BIOCHIMICA ET BIOPHYSICA ACTA - GENERAL SUBJECTS, ELSEVIER SCIENCE PUBLISHERS, NL, vol. 1790, no. 2, 1 February 2009 (2009-02-01), pages 141-146, XP025868654 ISSN: 0304-4165 [retrieved on 2008-11-05]
- YOSHIDA T ET AL: "A LIQUID-PHASE BINDING ANALYSIS FOR L-SELECTIN. A STRONG DEPENDENCY ON HIGHLY CLUSTERED SULFATE GROUPS" EUROPEAN JOURNAL OF BIOCHEMISTRY, BLACKWELL PUBLISHING, BERLIN, DE, vol. 222, no. 2, 1 January 1994 (1994-01-01), pages 703-709, XP000614583 ISSN: 0014-2956
- VASS J A ET AL: "Biotinylated L-selectin ligand analogs as cytochemical probes in cytospin preparations" FOLIA HISTOCHEMICA ET CYTOBIOLOGICA 1995 PL, vol. 33, no. 1, 1995, pages 39-41, XP008114481 ISSN: 0015-5586

## Description

### Background of the Invention

Numerous human degenerative diseases, including cardiovascular degenerative diseases, but also organ-specific degenerative diseases, involve circulating cell/vascular wall interactions. Selectins are important cell adhesion molecules, with high affinities for carbohydrate moieties. They play a prominent and critical role in the initial stages of circulating cellular components and vascular wall interactions by mediating leucocytes/platelet and leucocytes/endothelium interactions. Three types of selectins have been discovered so far: P-selectin, E-selectin and L-selectin. L-selectin is constitutively expressed on almost all circulating leukocytes. The expression of E-selectin is inducible on vascular endothelium upon activation by various mediators including cytokines and endotoxin. P-selectin is contained in intracytoplasmic granules and is rapidly translocated to platelet or endothelial surfaces after cell exposure to thrombin or histamine.

The P-, L- and E-selectins are structurally similar transmembrane proteins. They all possess large, highly glycosylated, extracellular domains, a single spanning transmembrane domain, and a small cytoplasmic tail. At their extracellular amino termini, they have a single calcium-dependent (or C-type) lectin domain (L) followed by an epidermal growth factor (EGF)-like domain (E) and several complement regulatory domains (C). Selectin-mediated cell adhesion results from calcium-dependent interactions of the amino-terminal lectin domain with a large variety of carbohydrate-presenting molecules on the surface of target cells. While the affinity of each of the selectins varies depending on the ligand, they all bind a specific tetrasaccharide carbohydrate structure known as sialyl Lewis X (SLe^{x}), which contains sialic acid and fucose residues.

Although selectin-mediated binding events play a critical role in normal physiological processes, selectins are also known to contribute to many pathologies. Such pathologies include clinical conditions that are associated with platelet activation and fibrin formation such as atherothrombotic diseases (E. Galkina et al., Curr. Drug Targets, 2007, 8: 1239-1248); clinical conditions associated with acute endothelial activation such as sepsis, brain ischemia, or ischemia-reperfusion (C.R. Calvey et al., J. Invest. Surg., 2007, 20: 71-85); clinical conditions associated with chronic endothelial activation such as hypertension, hyperlipidemia, obesity (S. Nishimura et al., J. Clin. Invest., 2008, 118: 710-721) or degenerative disorders of the cardiovascular system, the lung or the brain (M. Fisher, Rev. Neurol. Dis., 2008, 5 Suppl. 1: S4-S11; S.I. van Kasteren et al., Proc. Natl. Acad. Sci. USA, 2009, 106: 18-23); and clinical conditions associated with chronic focalized accumulation of leukocytes such as tertiary lymphoid neogenesis or autoimmune diseases. Selectin interactions can also mediate adhesive mechanisms involved in the metastasis of certain epithelial cancers (I.P Witz, Immunol. Lett., 2006, 104: 89-93; 1; S. Gout et al., Clin. Exp. Metastasis, 2008, 25: 335-344; L. Borsig, Expert Rev. Anticancer Ther., 2008, 8: 1247-1255).

Selectins are considered as potentially useful markers for the diagnosis of some of these pathologies. Numerous efforts are in progress to image selectins predominantly through Magnetic Resonance Imaging (MRI) (S. Bouty et al., Contrast Media Mol. Imaging, 2006, 1: 15-22), scintigraphy (G. Hairi et al., Ann. Biomed. Eng., 2008, 36: 821-830), and more recently using ultrasound (F.S. Villanueva et al., Nat. Clin. Pract. Cardiovasc. Med., 2008, 5: S26-S32). Most selectin imaging agents developed so far are anti-selectin antibodies (B.A. Kaufinan et al., Eur. Heart J., 2007, 28: 2011-2017; G. Hairi et al., Ann. Biomed. Eng., 2008, 36: 821-830; K. Licha et al., J. Biomed. Opt., 2005, 10: 41205; and P. Hauff et al., Radiology, 2004, 231: 667-673), selectin binding peptides (S.Gratz et al., Nucl. Med. Commun., 2001, 22:1003-1013) and sialyl Lewis X analogs or derivatives (S. Bouty et al., Contrast Media Mol. Imaging, 2006, 1: 15-22; F.S. Villanueva et al., Circulation, 2007, 115: 345-352). These imaging agents have been demonstrated to allow the *in vivo* non-invasive detection of selectins in inflammation, neurodegenerative disorders, cancer and thrombosis. However, they exhibit several disadvantages that will certainly preclude their industrial development and commercialization. Indeed, the preparation and purification of sialyl Lewis X-based imaging agents and of antibodies-based imaging agents is complex and very costly.

Therefore, there remains a need in the art for new approaches for the imaging and detection of circulating cell/vascular wall interactions allowing the non-invasive diagnosis and/or the preventive screening of diseases such as cardio/neurovascular pathologies, neurodegenerative disorders and cancer metastasis. Selectin imaging agents that are easy and relatively cheap to produce are particularly desirable.

### Summary of the Invention

The present invention relates to improved systems and strategies for the detection of selectins and the diagnosis of diseases and disorders characterized by undesirable or abnormal interactions mediated by selectins. In particular, the invention encompasses the recognition by the Applicants that fucoidans exhibit high affinity, specificity and/or selectivity for selectins. More specifically, the present Applicants have compared the interaction of P-selectin with several low molecular weight (LMW) polysaccharides: fucoidan, heparin and dextran sulfate. Using binding assay, mass spectrometry, surface plasmon resonance and flow cytometry on human platelets, they found that LMW fucoidan is the most efficient ligand of P-selectin (see Example 1). However, a less specific binding of fucoidan to fibrin moieties through hydrogen bonds is not excluded (K.H. Hsieh, Biochemistry, 1997, 36: 9381-9387). The Applicants also showed that LMW fucoidan radiolabelled with Technetium-99m (^{99m}Tc) allowed the *in vivo* detection of endocarditic vegetations, aneurismal and atrial thrombi in animal models (conditions associated with platelet-selectin exposition and fibrin formation) (see Example 2).

Accordingly, the present invention provides imaging agents comprising at least one fucoidan for the detection and imaging of selectins and for the diagnosis of diseases and disorders characterized by undesirable or abnormal expression of selectins.

More specifically, in one aspect, the present invention provides an imaging agent comprising at least one fucoidan moiety associated with at least one detectable moiety, wherein the at least one detectable moiety is detectable by planar scintigraphy (PS) or Single Photon Emission Computed Tomography (SPECT). Preferably, the imaging agent is selectin-targeted. More preferably, the at least one fucoidan moiety of the imaging agent binds at least one human selectin selected from the group consisting of P-selectin, L-selectin, and E-selectin with a dissociation constant of between about 0.1 nM and about 500 nM, preferably between about 0.5 nM and about 10 nM, more preferably between about 1 nM and about 5 nM.

The detectable moiety, which is detectable by planar scintigraphy (PS) or Single Photon Emission Computed Tomography (SPECT), is a radionuclide selected from the group consisting of technetium-99m (^{99m}Tc), gallium-67 (⁶⁷Ga), yttrium-91 (⁹¹Y), indium-111 (¹¹¹In), rhenium-186 (¹⁸⁶Re), and thallium-201 (²⁰¹Tl). In certain preferred embodiments, the detectable moiety is technetium-99m (^{99m}Tc). Another detectable moiety disclosed herein may comprise a metal-chelating moiety complexed to a detectable moiety. Another detectable moiety disclosed herein may be detectable by Positron Emission Tomography (PET). For example, the detectable moiety may be selected from the group consisting of carbon-11 (¹¹C), nitrogen-13 (¹³N), oxygen-15 (¹⁵O) and fluorine-18 (¹⁸F).

Another detectable moiety disclosed herein may be detectable by contrast-enhanced ultrasonography (CEUS). For example, the detectable moiety may be selected from the group consisting of acoustically active microbubbles and acoustically active liposomes.

Another detectable moiety disclosed herein may be detectable by Magnetic Resonance Imaging (MRI). For example, the detectable moiety may be selected from the group consisting of gadolinium III (Gd³⁺), chromium III (Cr³⁺), dysprosium III (Dy³⁺), iron III (Fe³⁺), europium (Eu³⁺), manganese II (Mn²⁺), and ytterbium III (Yb³⁺), preferably, the detectable moiety is gadolinium III (Gd³⁺). Alternatively, the detectable moiety may be an ultrasmall superparamagnetic iron oxide particle (USPIO).

Another detectable moiety disclosed herein may be detectable by fluorescence spectroscopy. For example, the detectable moiety may be selected from the group consisting of europium (Eu³⁺), quantum dots, Texas red, fluorescein isothiocyanate (FITC), phycoerythrin (PE), rhodamine, carboxycyanine, , Cy-3, Cy-5, Cy5.5, Cy7, DY-630, DY-635, DY-680, Atto 565 dyes, merocyanine, styryl dye, oxonol dye, BODIPY dyes, and analogues, derivatives or combinations of these molecules. In particular, the detectable moiety may be detectable by time-resolved fluorometry. For example, the detectable moiety may be europium (Eu³⁺).

An imaging agent may be detectable by more than one imaging technique and may therefore be used in multimodal imaging. For example, an imaging agent may be detectable by any suitable combination of imaging techniques selected from the group consisting of ultrasonography, Magnetic Resonance Imaging (MRI), Positron Emission Tomography (PET), Single Photon Emission Computed Tomography (SPECT), fluorescence spectroscopy, Computed Tomography, and X-ray radiography. Such an imaging agent may comprise at least one fucoidan moiety associated with at least one detectable moiety that is detectable by more than one imaging technique. Such an imaging agent may comprise at least one fucoidan moiety associated with a first detectable moiety and a second detectable moiety, wherein the first detectable moiety is detectable by a first detectable moiety and the second detectable moiety is detectable by a second detectable moiety.

In certain embodiments, the fucoidan moiety has an average molecular weight of about 2000 to about 8000 Da. In other embodiments, the fucoidan moiety has an average molecular weight of about 20,000 to about 70,000 Da. In yet other embodiments, the fucoidan moiety has an average molecular weight of about 100,000 to about 500,000 Da.

In another aspect, the present invention provides a pharmaceutical composition comprising an effective amount of at least one imaging agent of the invention, or a physiologically tolerable salt thereof, and at least one pharmaceutically acceptable carrier.

In a related aspect, the present invention provides for the use of an imaging agent according to the invention for the manufacture of a composition for the detection and/or imaging of selectins. The present invention also provides for the use of an inventive imaging agent for the manufacture of a composition for the diagnosis of a clinical condition associated with selectins.

In still another aspect, the present invention provides a selectin-targeted imaging agent for use in a method for diagnosing a clinical condition associated with selectins in a patient, said method comprising steps of: administering to the patient an effective amount of an imaging agent or a pharmaceutical composition thereof; and detecting any selectin bound to the imaging agent using an imaging technique.

In a related aspect, the present invention provides an imaging agent disclosed herein for use in an *in vivo* method of diagnostic of clinical conditions associated with selectins.

Examples of clinical conditions that can be diagnosed using an imaging agent and/or a method of the invention are members of the group consisting of thrombosis, myocardial ischemia/reperfusion injury, stroke and ischemic brain trauma, neurodegenerative disorders, tumor metastasis and tumor growth, and rheumatoid arthritis.

In yet another aspect, the present invention provides a method for detecting the presence of abnormal selectins in a biological system, the method comprising steps of: contacting the biological system with an effective amount of an imaging agent or a pharmaceutical composition thereof; and detecting any selectin bound to the imaging agent using an imaging technique. The biological sample may be a cell, a biological fluid or a biological tissue.

In a related aspect, the present invention provides an imaging agent disclosed herein for use in an *in vitro* method of diagnostic of clinical conditions associated with selectins.

In certain embodiments, the biological sample originates from a patient suspected of having a clinical condition associated with selectins, and the method is used to diagnose the clinical condition.

In other embodiments, the biological sample originates from a patient who has received a treatment for a clinical condition associated with selectins, and the method is used to monitor the response of a patient to the treatment.

In yet still another aspect, the present invention provides kits for the diagnosis of a clinical condition associated with selectins in a patient or for the detection of abnormal selectins in a biological tissue, the kit comprising a fucoidan moiety, a detectable moiety, and instructions for preparing a selectin-targeted imaging agent described herein, wherein the detectable moiety is a short-lived radionuclide selected from the group consisting of technetium-99m (^{99m}Tc), gallium-67 (⁶⁷Ga), yttrium-91 (⁹¹Y), indium-111 (¹¹¹In), rhenium-186 (¹⁸⁶Re), and thallium-201 (²⁰¹T1).

The kit may further comprise instructions for diagnosing the clinical condition using the selectin-targeted imaging agent.

These and other objects, advantages and features of the present invention will become apparent to those of ordinary skill in the art having read the following detailed description of the preferred embodiments.

### Brief Description of the Drawing

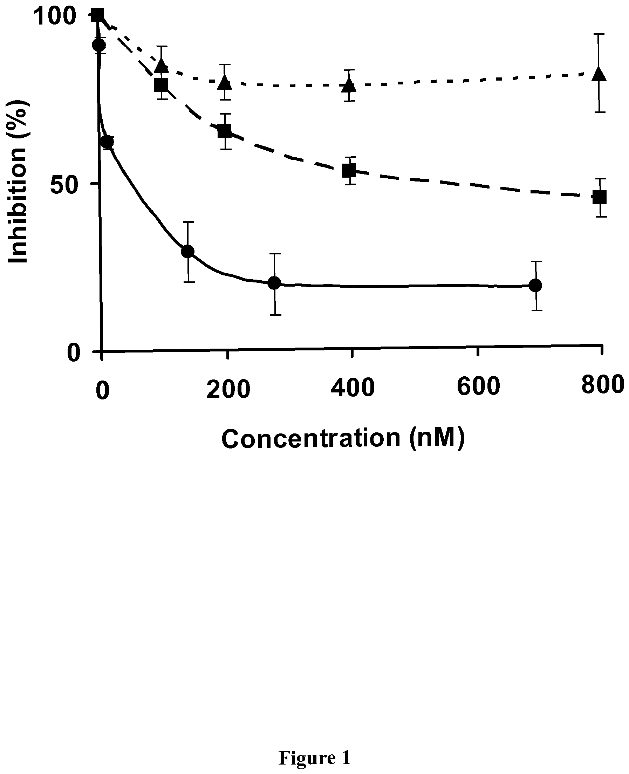

**Figure 1** **is** a graph showing inhibition of SLe^{x}/P-selectin binding by sulfated polysaccharides. Binding of SLe^{x} polyacrylamide-biotin to P-selectin immobilized onto a microtiter plate was quantified by streptavidin-peroxidase complexation and peroxidase reaction recorded at 405 nm in the presence of increasing concentrations of dextran sulfate (▲), heparin (■) and fucoidan (●), as described in Example 1. The results of a representative experiment are shown [mean ± SD (n≥3)].

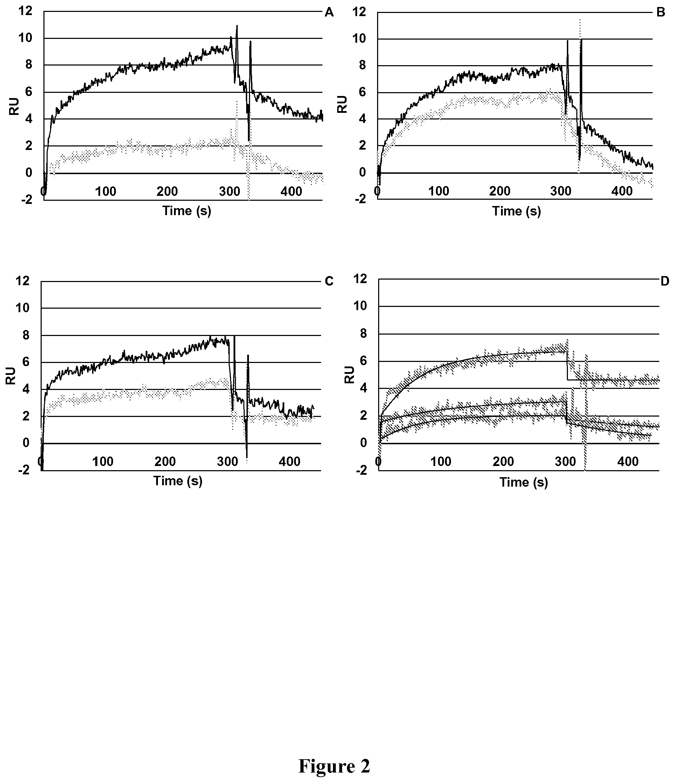

**Figure 2** is a set of representative sensorgrams showing the association and dissociation profiles of sulfated polysaccharides on immobilized IgG or P-selectin. Fucoidan **(A),** heparin **(B)** and dextran sulfate **(C)** were injected over SPR CM5 sensorchips on which were immobilized goat anti-human Fc IgG (grey recording, non specific control) plus P-selectin/Fc chimera (black recording). Kinetic studies were performed at a flow rate of 30 µL/min. Representative sensorgrams in resonance units (RU) are overlaid at a similar 1 µM concentration for all LMW sulfated polysaccharides. Dissociation constants were calculated using a 1:1 Langmuir binding model plot **(D)** for the specific binding of P-selectin with fucoidan (upper curve), heparin (lower curve) or dextran sulfate (middle curve). Non-specific binding on IgG was observed for each of the polysaccharides.

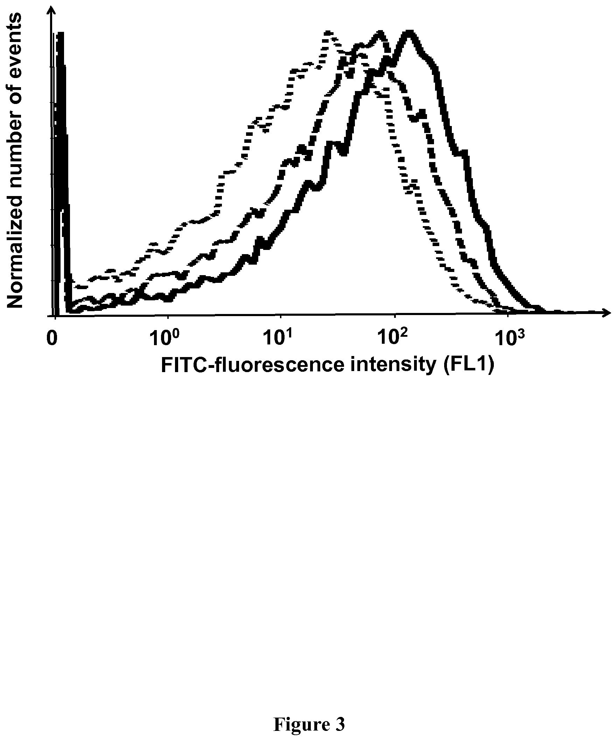

**Figure 3** is a graph showing the binding of FITC-coupled LMW fucoidan to human platelets in whole blood. FITC-coupled LMW fucoidan at 140 µM (1 mg/mL) was incubated for 20 minutes at room temperature with citrated human blood diluted 10 times in PBS. Activation of platelets was induced with 2.5 µM ADP (medium activator; dotted line) or 200 µM TRAP (strong activator; full line). Platelets were identified by their side and forward scatter and their positivity for a fluorolabeled specific platelet antibody in flow cytometry. Binding of FITC-coupled LMW fucoidan to platelets was detected on the FL1 channel. Similar results were obtained using two other donors.

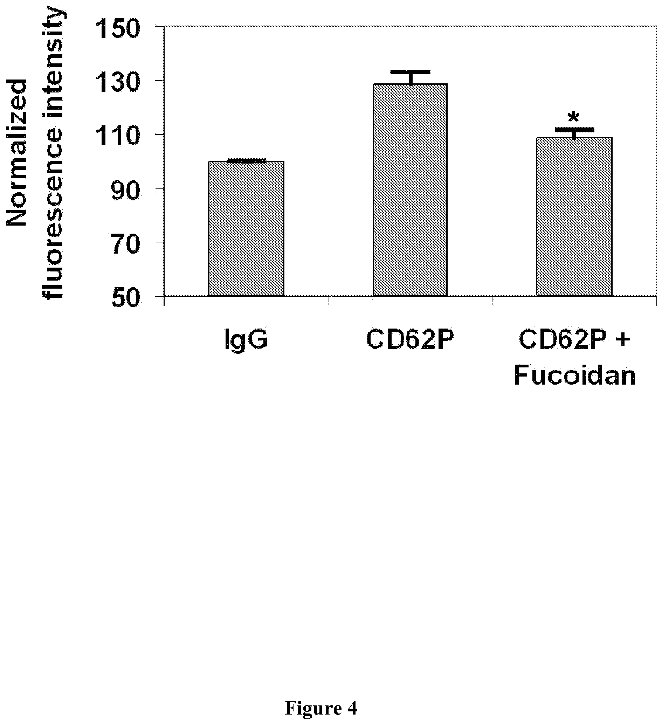

**Figure 4** is a graph showing the binding inhibition of labeled CD62P antibody to platelets in the presence of LMW fucoidan. CD62P antibody was incubated in the presence or in the absence of non-labeled LMW fucoidan, as described in Example 1. Activation of platelets was induced by 200 µM TRAP. Platelets were identified by their side and forward scatter and their positivity for a fluorolabeled specific platelet antibody in flow cytometry. Binding to activated platelets of non-relevant PC5-labeled IgG antibody is reported for comparison. The binding of PC5-labeled CD62P antibody to platelets, observed on the FL4 channel, significantly decreased in the presence of LMW fucoidan. Values of mean fluorescence intensity (MFI) were normalized to the value obtained by incubation with non-relevant IgG alone. *p<0.05 between data with CD62P alone with Student's t-test.

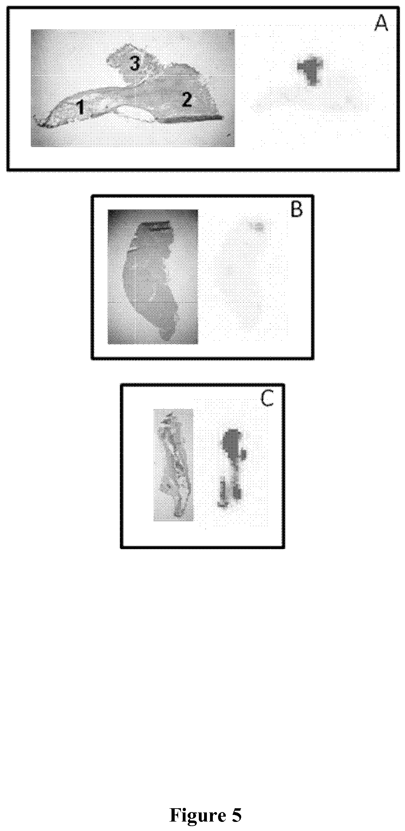

**Figure 5** shows histology (left) and autoradiography (right) sections of hearts in rat model of left endocarditis with aortic valve vegetations. **(A)** One histologic section vegetation is restricted to the valve (3) whereas the aorta (1) and the sub-valvular myocardium (2) were normal. On the autoradiography, the signal from ^{99m}Tc-labelled fucoidan, injected *in vivo,* is exactly co-localized with the valvular vegetation. **(B)** A negative control of a myocardium without vegetation gives the background in autoradiography. **(C)** On the autoradiography, the signal from ^{99m}Tc-labelled fucoidan is exactly co-localized with the fibrinoid cuff surrounding the catheter.

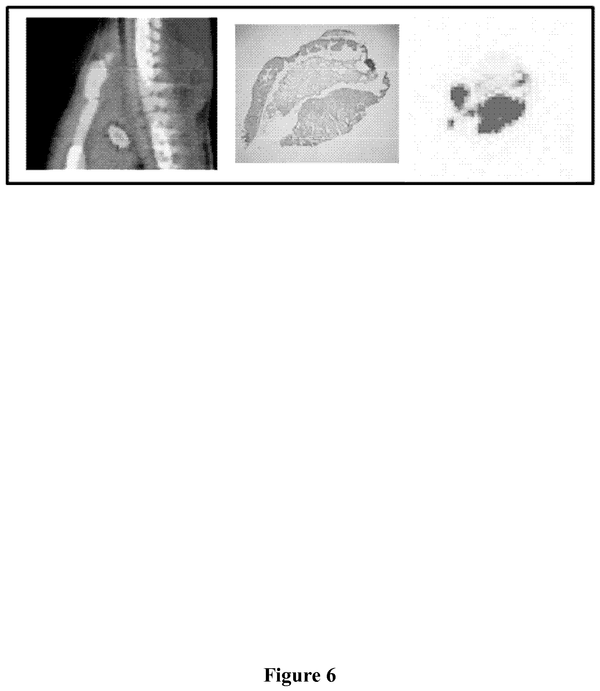

**Figure 6** shows tomography-SPECT *in vivo* imaging (left), histology (middle) and autoradiography (right) section in rat model of atrial thrombus. The tomography-SPECT shows retention of ^{99m}Tc-labelled fucoidan in the rat left atrium. The histology results show that there is fibrinous thrombus in the atrial lumen with muscle on both sides. On the autoradiography, the signal from ^{99m}Tc-labelled fucoidan is localized in the myocardium facing the thrombus.

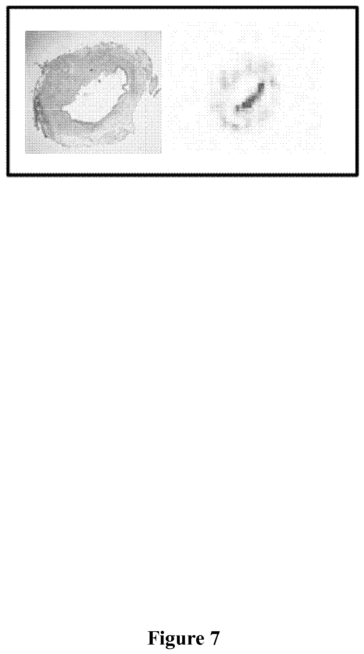

**Figure 7** shows histology (left) and autoradiography (right) sections of an abdominal aortic aneurysm in a rat model of aneurismal thrombus. On the autoradiography, the signal from ^{99m}Tc-labelled fucoidan is localized at the lumen/vessel wall interface where a thin thrombus (blue) is localized on the histology picture.

### Definitions

Throughout the specification, several terms are employed that are defined in the following paragraphs.

As used herein, the term "***selectin***" has its art understood meaning and refers to any member of the family of carbohydrate-binding, calcium-dependent cell adhesion molecules that are constitutively or inductively present on the surface of leukocytes, endothelial cells or platelets. The term *"E-selectin",* as used herein, has its art understood meaning and refers to the cell adhesion molecule also known as SELE; CD62E; ELAM; ELAM1; ESEL; or LECAM2 (Genbank Accession Numbers for human E-selectin: NM_000450 (mRNA) and NP_000441 (protein)). As used herein, the term "***L***-***selectin***" has its art understood meaning and refers to the cell adhesion molecule also known as SELL; CD62L; LAM-1; LAM1; LECAM1; LNHR; LSEL; LYAM1; Leu-8; Lyam-1; PLNHR; TQ1; or hLHRc (Genbank Accession Numbers for human L-selectin: NM_000655 (mRNA) and NP_000646 (protein)). The term "***P***-***selectin***", as used herein, has its art understood meaning and refers to the cell adhesion molecule also known as a SELP; CD62; CD62P; FLJ45155; GMP140; GRMP; PADGEM; or PSEL (Genbank Accession Numbers for human P-selectin: NM_003005 (mRNA) and NP_002996 (protein)).

As used herein, the term ***"imaging agent"*** refers to a compound that can be used to detect specific biological elements (*e.g.*, biomolecules) using imaging techniques. Imaging agents of the invention are molecules comprising at least one fucoidan moiety associated with at least one detectable moiety. Imaging agents of the present invention can be used to detect selectins in *in vitro* and *ex vivo* biological systems as well as in subjects.

The term "***fucoidan moiety***" refers to any fucoidan entity exhibiting high affinity, specificity and/or selectivity for selectins. In the context of the present invention, when a fucoidan moiety is part of a molecule (*e.g.*, an imaging agent), it confers its specificity/selectivity/affinity property to the molecule, and the molecule becomes "***select**-**in***-***targeted"*** (*i.e.,* the molecule specifically and/or efficiently interacts with and/or binds to selectins).

The terms "***binding affinity***" and "***affinity***" are used herein interchangeably and refer to the level of attraction between molecular entities. Affinities can be expressed quantitatively as a dissociation constant (K_{d} or K_{D}), or its inverse, the association constant (Kₐ or K_{A}).

The term "***detectable moiety***", as used herein refers to any entity which, when part of a molecule, allows visualization of the molecule, for example using imaging techniques.

The terms ***"pathological condition associated with selectins***", "***disease associated** with **selectins***" and "***disorder associated with selectins***" are used herein interchangeably. They refer to any disease condition characterized by undesirable or abnormal selectin-mediated interactions. Such conditions include, for example, disease conditions associated with or resulting from the homing of leukocytes to sites of inflammation, the normal homing of lymphocytes to secondary lymph organs, the interaction of platelets with activated endothelium, platelet-platelet and platelet-leukocyte interactions in the blood vascular compartment, and the like. Examples of such disease conditions include, but are not limited to, tissue transplant rejection, platelet-mediated diseases (e.g., atherosclerosis and clotting), hyperactive coronary circulation, acute leukocyte-mediated lung injury (e.g., adult respiratory distress syndrome - ARDS), Crohn's disease, inflammatory diseases (*e.g.*, inflammatory bowel disease), autoimmune diseases (e.g., multiple sclerosis, myasthenia gravis), infection, cancer (including metastasis), thrombosis, wounds and wound-associated sepsis, burns, spinal cord damage, digestive tract mucous membrane disorders (*e.g.*, gastritis, ulcers), osteoporosis, rheumatoid arthritis, osteoarthritis, asthma, allergy, psoriasis, septic shock, stroke, nephritis, atopic dermatitis, frostbite injury, adult dysponoea syndrome, ulcerative colitis, systemic lupus erythrematosis, diabetes and reperfusion injury following ischemic episodes.

As used herein, the term "***subject***" refers to a human or another mammal (e.g., mouse, rat, rabbit, hamster, dog, cat, cattle, swine, sheep, horse or primate). In many embodiments, the subject is a human being. In such embodiments, the subject is often referred to as an ***"individual",*** or a ***"patient"*** if the subject is afflicted with a disease or clinical condition. The terms "subject", "individual" and "patient" do not denote a particular age, and thus encompass adults, children and newborns.

The term "***biological sample"*** is used herein in its broadest sense. A biological sample is generally obtained from a subject. A sample may be of any biological tissue or fluid that can produce and/or contain selectins. Frequently, a sample will be a "clinical sample", *i.e.,* a sample derived from a patient. Such samples include, but are not limited to, bodily fluids which may or may not contain cells, *e.g.,* blood, urine, saliva, cerebrospinal fluid (CSF), cynovial fluid, tissue or fine needle biopsy samples, and archival samples with known diagnosis, treatment and/or outcome history. Biological samples may also include sections of tissues such as frozen sections taken for histological purposes. The term "biological sample" also encompasses any material derived by processing a biological sample. Derived materials include, but are not limited to, cells (or their progeny) isolated from the sample, proteins or other molecules extracted from the sample. Processing of a biological sample may involve one or more of: filtration, distillation, extraction, concentration, inactivation of interfering components, addition of reagents, and the like.

The term "***effective amount***", when used herein in reference to a selectin-targeted imaging agent of the invention, or a pharmaceutical composition thereof, refers to any amount of the imaging agent, or pharmaceutical composition, which is sufficient to fulfill its intended purpose(s) (*e.g.*, the purpose may be the detection and/or imaging of selectins present in a biological system or in a subject, and/or the diagnosis of a disease associated with selectins).

A "***pharmaceutical composition",*** as used herein, is defined as comprising at least one selectin-targeted imaging agent, or a physiological tolerable salt thereof, and at least one pharmaceutically acceptable carrier.

The term "***physiologically tolerable salt***" refers to any acid addition or base addition salt that retains the biological activity and properties of the free base or free acid, respectively, and that is not biologically or otherwise undesirable. Acid addition salts are formed with inorganic acids (*e.g*., hydrochloric, hydrobromic, sulfuric, nitric, phosphoric acids, and the like); and organic acids (*e.g.*, acetic, propionic, pyruvic, maleic, malonic, succinic, fumaric, tartaric, citric, benzoic, mandelic, methanesulfonic, ethanesulfonic, *p-*toluenesulfonic, salicylic acids, and the like). Base addition salts can be formed with inorganic bases (e.g., sodium, potassium, lithium, ammonium, calcium, magnesium, zinc, aluminum salts, and the like) and organic bases (*e.g*., salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethyl-aminoethanol, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins, and the like).

As used herein, the term "***pharmaceutically acceptable carrier"*** refers to a carrier medium which does not interfere with the effectiveness of the biological activity of the active ingredients and which is not excessively toxic to the hosts at the concentrations at which it is administered. The term includes solvents, dispersion media, coatings, antibacterial and antifungal agents, isotonic agents, adsorption delaying agents, and the like. The use of such media and agents for pharmaceutically active substances is well known in the art (see, for example, Remington's Pharmaceutical Sciences, E.W. Martin, 18th Ed., 1990, Mack Publishing Co., Easton, PA).

The term *"treatment"* is used herein to characterize a method or process that is aimed at (1) delaying or preventing the onset of a disease or condition (*e.g.*, a selectin-associated state or condition); (2) slowing down or stopping the progression, aggravation, or deterioration of the symptoms of the state or condition; (3) bringing about amelioration of the symptoms of the state or condition; and/or (4) curing the state or condition. A treatment may be administered prior to the onset of the disease, for a prophylactic or preventive action. Alternatively or additionally, a treatment may be administered after initiation of the disease or condition, for a therapeutic action.

The terms ***"approximately"*** and ***"about",*** as used herein in reference to a number, generally include numbers that fall within a range of 10% in either direction of the number (greater than or less than the number) unless otherwise stated or otherwise evident from the context (except where such a number would exceed 100% of a possible value).

### Detailed Description of Certain Preferred Embodiments

As mentioned above, the present invention is directed to the use of fucoidans for the imaging of selectins and the diagnosis of pathophysiological conditions associated with selectins. In particular, the invention encompasses imaging agents, kits and strategies for specifically detecting the presence of selectins *in vitro, ex vivo* as well as *in vivo* using imaging techniques.

### I - Selectin-targeted Imaging Agents

In one aspect, the invention relates to a new class of imaging agents that have high affinity and specificity for selectins. More specifically, selectin-targeted imaging agents are provided that comprise at least one fucoidan moiety associated with at least one detectable moiety.

### Fucoidan Moieties

Fucoidans (also called fucosans or sulfated fucans) are sulfated polysaccharides with a wide spectrum of biological activities, including anticoagulant, antithrombotic, antivirus, antitumor, immunomodulatory, anti-inflammatory, and antioxidant activities (B. Li et al., Molecules, 2008, 13: 1671-1695; D. Logeart et al., J. Biomed. Mater Res., 1996, 30: 501-508). Fucoidans are found mainly in various species of brown seaweed (B. Li et al., Molecules, 2008, 13: 1671-1695; M. Kusaykin et al., Biotechnol. J., 2008, 3: 904-915). Variant forms of fucoidans have also been found in marine animal species, including the sea cucumber. Thus, compared to other sulfated polysaccharides, fucoidans are widely available from various kinds of cheap sources, and easily obtained using methods of extraction known in the art (C. Colliec et al., Phytochemistry, 1994, 35(3): 697-700). These methods of extraction generally yield fucoidans with molecular weights in the 70-800 kDa range. Processes have also been developed to prepare low molecular weight fucoidans from high molecular weight fucoidans, *e.g.*, lower than about 20 kDa

(EP 0 403 377B, US Pat. No. 5,321,133), or lower than about 10 kDa (EP 0 846 129 B; U.S. Pat. No. 6,028,191; A. Nardella et al., Carbohydr. Res., 1996, 289: 201-208).

Fucoidans are α-1,2- or α-1,3- linked L-fucose polymers that are sulfated on position 4 and position 2 or 3 following the glycosidic linkage. However, besides fucose and sulfate residues, fucoidans also contain other monosaccharides (e.g., mannose, galactose, glucose, xylose, etc) and uronic acid groups. It is known in the art that the structure of fucoidans from different brown algae varies from species to species. Furthermore, the structure of fucoidans can also be chemically modified. For example, methods have been developed to increase the percentage of sulfate groups of fucoidans in order to obtain oversulfated fucoidans or oversulfated fucoidan fragments (T. Nishino et al., Carbohydr. Res., 1992, 229: 355-362; S. Soeda et al., Thromb. Res., 1993, 72: 247-256).

Fucoidan moieties suitable for use in the present invention are fucoidan moieties that have some degree of attraction for selectins and can play a targeting role when comprised in an imaging agent. Preferably, fucoidan moieties are stable, non-toxic entities that retain their affinity/specificity/selectivity properties under *in vitro* and *in vivo* conditions. In preferred embodiments, fucoidan moieties exhibit high affinity and specificity for selectins, *i.e.,* they specifically and efficiently interact with, bind to, or associate with selectins. Suitable fucoidan moieties include fucoidans that exhibit affinity and specificity for only one of the selectins *(i.e.,* for L-selectin, E-selectin or P-selectin) as well as fucoidans that exhibit affinity and specificity for more than one selectin, including those moieties which can efficiently interact with, bind to or associate with all three selectins. Preferably, the interaction between a selectin and a fucoidan moiety within an imaging agent is strong enough for at least the time necessary to detect the selectin using an imaging technique. In certain embodiments, a suitable fucoidan moiety interacts with a selectin with a dissociation constant (K_{d}) between about 0.1 nM and about 500 nM, preferably between about 0.5 nM and about 10 nM, more preferably between about 1 nM and about 5 nM.

The design of an inventive imaging agent will be dictated by its intended purpose(s) and the properties that are desirable in the particular context of its use. Thus, fucoidan moieties will be chosen based on their known, observed or expected, properties. For example, in embodiments where an imaging agent of the invention is to be used in the diagnosis of neurodegenerative disorders characterized by undesirable or abnormal selectin-mediated interactions in the brain, the imaging agent will preferably be capable of crossing the blood-brain barrier. Therefore, such an imaging agent will preferably contain a fucoidan moiety of low molecular weight (*e.g.*, 2-8 kDa or lower than 5 kDa). In contrast, an imaging agent containing a fucoidan moiety of high molecular weight will be more suited for situations in which the agent is to be used to image selectins in the vascular system. Indeed, because of its high molecular weight, the imaging agent will not be able to easily diffuse and will therefore more likely remain within the vascular system, thereby allowing a more selective targeting of the system of interest.

A fucoidan moiety of high molecular weight can also have the advantage of being able to carry a high number of detectable moieties, thus increasing the sensibility of the imaging agent *(i.e.,* allowing the detection of lower concentrations of selectins). In addition to their molecular weight, fucoidan moieties may be selected based on their sulfate content. By varying the sulfate content (either by selection of naturally-occurring fucoidans or by chemical modification), it may be possible to modulate the specificity of the fucoidan moiety (and corresponding imaging agent) for one of the selectins (L-selectin, E-selectin or P-selectin). It is known, for example, that binding to P- and E-selectins increases with the presence of sulfate groups on the ligand (T.V. Pochechueva et al., Bioorganic & Medicinal Chemistry Letters, 2003, 13: 1709-1712).

Alternatively or additionally, a fucoidan moiety may be selected based on its structure and, in particular, based on the presence of at least one functional group that can be used (or that can be easily chemically converted to a different functional group that can be used) to associate the fucoidan moiety to a detectable moiety. Examples of suitable functional groups include, but are not limited to, carboxy groups, thiols, amino groups (preferably primary amines), and the like.

### Detectable Moieties

Detectable moieties disclosed herein are entities that are detectable by imaging techniques such as ultrasonography, Magnetic Resonance Imaging (MRI), Positron Emission Tomography (PET), Single Photon Emission Computed Tomography (SPECT), fluorescence spectroscopy, Computed Tomography, X-ray radiography, or any combination of these techniques. Preferably, detectable moieties are stable, non-toxic entities which, when part of a selectin-targeted imaging agent, retain their properties under *in vitro* and *in vivo* conditions.

*Radioactive Imaging Moieties.* The selectin-targeted imaging agent disclosed herein may be designed to be detectable by a nuclear medicine imaging techniques such as planar scintigraphy (PS), Positron Emission Tomography (PET) and Single Photon Emission Computed Tomography (SPECT). In such cases, the imaging agent of the invention comprises at least one fucoidan moiety associated with at least one radionuclide *(i.e.,* a radioactive isotope) as defined in the claims.

SPECT and PET have been used to detect tumors, aneurysms, irregular or inadequate blood flow to various tissues, blood cell disorders, and inadequate functioning of organs, such as thyroid and pulmonary function deficiencies. Both techniques acquire information on the concentration of radionuclides introduced into a biological sample or a patient's body. PET generates images by detecting pairs of gamma rays emitted indirectly by a positron-emitting radionuclide. A PET analysis results in a series of thin slice images of the body over the region of interest (*e.g.*, brain, breast, liver). These thin slice images can be assembled into a three dimensional representation of the examined area. However, there are only few PET centers because they must be located near a particle accelerator device that is required to produce the short-lived radioisotopes used in the technique. SPECT is similar to PET, but the radioactive substances used in SPECT have longer decay times than those used in PET and emit single instead of double gamma rays. Although SPECT images exhibit less sensitivity and are less detailed than PET images, the SPECT technique is much less expensive than PET and offers the advantage of not requiring the proximity of a particle accelerator. Planar scintigraphy (PS) is similar to SPECT in that it uses the same radionuclides. However, PS only generates 2D-information.

Thus, the detectable moiety in an imaging agent disclosed herein may be a radionuclide detectable by PET. Examples of such radionuclides include carbon-11 (¹¹C), nitrogen-13 (¹³N), oxygen-15 (¹⁵O) and fluorine-18 (¹⁸F).

In the present invention, the detectable moiety is a radionuclide detectable by planar scintigraphy or SPECT. Examples of such radionuclides include technetium-99m (^{99m}Tc), gallium-67 (⁶⁷Ga), yttrium-91 (⁹¹Y), indium-111 (¹¹¹In), rhenium-186 (¹⁸⁶Re), and thallium-201 (²⁰¹T1). Preferably, the radionuclide is technetium-99m (^{99m}Tc). Over 85% of the routine nuclear medicine procedures that are currently performed use radiopharmaceutical methodologies based on ^{99m}Tc.

*MRI Imaging Moieties.* The selectin-targeted imaging agent disclosed herein may be designed to be detectable by Magnetic Resonance Imaging (MRI). MRI, which is an application of Nuclear Magnetic Resonance (NMR), has evolved into one of the most powerful non-invasive techniques in diagnostic clinical medicine and biomedical research. It is widely used as a non-invasive diagnostic tool to identify potentially maleficent physiological anomalies, to observe blood flow or to determine the general status of the cardiovascular system. MRI has the advantage (over other high-quality imaging methods) of not relying on potentially harmful ionizing radiation.

Thus, the imaging agents disclosed herein may comprise at least one fucoidan moiety associated with at least one paramagnetic metal ion. Examples of paramagnetic metal ions detectable by MRI are gadolinium III (Gd³⁺), chromium III (Cr³⁺), dysprosium III (Dy³⁺), iron III (Fe³⁺), manganese II (Mn²⁺), and ytterbium III (Yb³⁺), preferably gadolinium III (Gd³⁺). Gadolinium is an FDA-approved contrast agent for MRI.

The imaging agents disclosed herein may comprise at least one fucoidan moiety associated with at least one ultrasmall superparamagnetic iron oxide (USPIO) particle. USPIO particles are currently under investigation as contrast agents for imaging human pathologies (C. Corot et al., Adv. Drug Deliv. Rev., 2006, 56: 1472-1504). They are composed of a crystalline iron oxide core containing thousands of iron atoms which provide a large disturbance of the Magnetic Resonance signal of surrounding water. In contrast to other types of nanoparticles such as quantum dots (currently under investigation as extremely sensitive fluorescent probes), USPIO particles exhibit a very good biocompatibility. Chemical coating of USPIO particles is required to ensure their dispersion in biological media. The presence of an appropriate coating may also result in a decrease in the clearance of the particles ("stealth" effect) and may provide a means to bind these particles to molecules that are able to target a specific tissue (R. Weissleder et al., Magn. Reson. Q, 1992, 8: 55-63). Polysaccharides, such as dextran and its carboxymethylated derivatives, are currently used as coatings. USPIO particles may be coated with fucoidan moieties and the resulting imaging agents may be used to detect selectins by MRI. Such imaging agents may find applications in the diagnosis of cardiovascular pathologies associated with selectins. Indeed, with a radius of about 15 nm, USPIO particles are likely to diffuse only weakly outside the vascular space with the exception of more permeable pathological vascular tissues such as atherosclerotic walls. Therefore, they constitute a good blood pool agent (J. Bremerich et al., Eur. Radiol., 2007, 17: 3017-3024).

USPIO particles are known in the art and have been described (see, for example, J. Petersein et al., Magn. Reson. Imaging Clin. Am., 1996, 4: 53-60; B. Bonnemain, J. Drug Target, 1998, 6: 167-174; E.X. Wu et al., NMR Biomed., 2004, 17: 478-483; C. Corot et al., Adv. Drug Deliv. Rev., 2006, 58: 1471-1504; M. Di Marco et al., Int. J. Nanomedicine, 2007, 2: 609-622). USPIO particles are commercially available, for example, from AMAG Pharmaceuticals, Inc. under the tradenames Sinerem® and Combidex^{®}.

*Contrast-Enhanced Ultrasonography Imaging Moieties.* The selectin-targeted imaging agent disclosed herein may be designed to be detectable by contrast-enhanced ultrasonography (CEUS). Ultrasound is a widespread technology for the screening and early detection of human diseases. It is less expensive than MRI or scintigraphy and safer than molecular imaging modalities such as radionuclide imaging because it does not involve radiation.

Thus, the imaging agents disclosed herein may comprise at least one fucoidan moiety associated with at least one acoustically active (gas-filled) microbubble. A variety of acoustically active microbubbles may be used (A.L. Klibanov, Bioconj. Chem., 2005, 16: 9-17; J.R. Lindner, Nat. Rev. Drug Discov., 2004, 3: 527-532; M. McCulloch et al., J. Am. Soc. Echocardiogr., 2000, 13: 959-967; A.M. Takalkar et al., J. Contr. Release, 2004, 96: 473-482; G.E. Weller et al., Biotechnol. Bioeng., 2005, 92: 780-788).

Generally, such microbubbles are comprised of a gas core and a shell. The gas core is the most important part of the microbubble because it allows detection. When gas bubbles are caught in an ultrasonic frequency field, they compress, oscillate and reflect a characteristic echo, which generates a strong and unique sonogram in CEUS. Gas cores can be composed of air, or heavy gases such as perfluorocarbon or nitrogen. Microbubbles with heavy gas-cores are likely to last longer in the circulation compared to microbubbles with air-comprising cores. The shell material determines how easily the microbubble is taken up by the immune system. A microbubble with a shell made of a more hydrophilic material tends to be taken up more easily by the immune system, while a more hydrophobic shell material tends to increase the microbubble residence time in the circulation, thus increasing the time available for contrast imaging. Microbubbles shells may be made of albumin, galactose, lipids or polymers (J.R. Lindner, Nat. Rev. Drug Discov., 2004, 3: 527-532). Regardless of the shell or gas core composition, microbubble size is fairly uniform. Their diameter is generally in the 1-4 micrometer range. Therefore, they are smaller than red blood cells, allowing them to flow easily through the circulation as well as the microcirculation (F.S. Vallanueva et al., Nat. Clin. Pract. Cardiovasc. Med., 2008, 5 Suppl. 2: S26-S32).

The imaging agent disclosed herein may comprise at least one fucoidan moiety associated with at least one acoustically active lipid particle *(i.e.,* a gas-filled liposome). A variety of acoustically active lipid particles are known in the art and may be used in the practice of the present invention (H. Alkan-Onyuksel et al., J. Pharm. Sci., 1996, 85: 486-490; S.M. Demos et al., J. Am. Coll. Cardiol., 1999, 33: 867-875; S.L. Huang et al., J. Pharm. Sci., 2001, 90: 1917-1926; S.L. Huang et al., J. Ultrasound Med., 2002, 28: 339-348; A. Hamilton et al., Circulation, 2002, 105: 2772-2778).

*Fluorescence Imaging Moieties.* The selectin-targeted imaging agent disclosed herein may be designed to be detectable by fluorescence spectroscopy. In such cases, the imaging agents may comprise at least one fucoidan moiety associated with at least one fluorescent moiety.

Suitable optical properties of fluorescent moieties include high molecular absorption coefficient, high fluorescence quantum yield, and photostability. Preferred fluorescent moieties exhibit absorption and emission wavelengths in the visible *(i.e.,* between 400 and 700 nm) or the near infra-red *(i.e.,* between 700 and 950 nm). Selection of a particular fluorescent moiety will be governed by the nature and characteristics of the illumination and detection systems used in the diagnostic method. *In vivo* fluorescence imaging uses a sensitive camera to detect fluorescence emission from fluorophores in whole-body living mammals. To overcome the photon attenuation in living tissue, fluorophores with emission in the near-infrared (NIR) region are generally preferred (J. Rao et al., Curr. Opin. Biotechnol., 2007, 18: 17-25). The list of NIR probes continues to grow with the recent addition of fluorescent organic, inorganic and biological nanoparticles. Recent advances in imaging strategies and reporter techniques for in vivo fluorescence imaging include novel approaches to improve the specificity and affinity of the probes, and to modulate and amplify the signal at target sites for enhanced sensitivity. Further emerging developments are aiming to achieve high-resolution, multimodality and lifetime-based *in vivo* fluorescence imaging.

Numerous fluorescent moieties with a wide variety of structures and characteristics are suitable. Suitable fluorescent labels include, but are not limited to, quantum dots *(i.e.,* fluorescent inorganic semiconductor nanocrystals) and fluorescent dyes such as Texas red, fluorescein isothiocyanate (FITC), phycoerythrin (PE), rhodamine, fluorescein, carbocyanine, Cy-3™ and Cy-5™ *(i.e.,* 3- and 5-N,N'-diethyltetra-methylindodicarbocyanine, respectively), Cy5.5, Cy7, DY-630, DY-635, DY-680, and Atto 565 dyes, merocyanine, styryl dye, oxonol dye, BODIPY dye *(i.e.,* boron dipyrromethene difluoride fluorophore), and analogues, derivatives or combinations of these molecules.

The detectable moiety disclosed herein be detectable by time-resolved fluorometry. For example, the detectable moiety is europium (Eu³⁺).

As will be understood by one skilled in the art, the selection of a particular type of detectable moiety in the design of a selectin-targeted imaging agent will be dictated by the intended purpose of the imaging agent as well as by the imaging technique to be used in the detection.

An imaging agent may be designed to be detectable by more than one imaging technique, for example by a combination of MRI-PET, MRI-SPECT, fluorescence-MRI, X-ray radiography-scintigraphy, and the like. Multimodal imaging provides different types of information about biological tissues, such as both structural and functional properties. Thus, for example, an imaging agent may comprise at least one fucoidan moiety associated with at least one detectable moiety that is detectable by more than one imaging technique. Examples of such detectable moieties include, but are not limited to, europium, which is fluorescent and detectable by MRI; and luminescent hybrid nanoparticles with a paramagnetic Gd₂O₃ core that are developed as contrast agents for both *in vivo* fluorescence and MRI (J.L; Bridot et al., J. Am. Chem. Soc., 2007, 129: 5076-5084) Alternatively, an imaging agent may comprise at least one fucoidan moiety associated with a first detectable moiety and a second detectable moiety, wherein the first detectable moiety is detectable by a first imaging technique and the second detectable moiety is detectable by a second imaging technique. A large variety of imaging agents with double detectability may thus be obtained. The simultaneous use of two different imaging agents *(i.e.,* of a first imaging agent detectable by a first imaging technique and a second imaging agent detectable by a second imaging technique) is also contemplated.

### Synthesis of Selectin-Targeted Imaging Agents

The imaging agents described herein may be prepared by any synthetic method known in the art, the only requirement being that, after reaction, the fucoidan moiety and detectable moiety retain their affinity and detectability property, respectively. The fucoidan and detectable moieties may be associated in any of a large variety of ways. Association may be covalent or non-covalent. When the association is covalent, the fucoidan and detectable moieties may be bound to each other either directly or indirectly (e.g., through a linker). When the detectable moiety is a metal entity, the fucoidan moiety may be associated to the detectable metal entity *via* a metal-chelating moiety.

More specifically, in certain embodiments, the fucoidan moiety and detectable moiety are directly covalently linked to each other. The direct covalent binding can be through an amide, ester, carbon-carbon, disulfide, carbamate, ether, thioether, urea, amine or carbonate linkage. The covalent binding can be achieved by taking advantage of functional groups present on the fucoidan moiety and detectable moieties. Suitable functional groups that can be used to attach the two moieties together include, but are not limited to, amines (preferably primary amines), anhydrides, hydroxy groups, carboxy groups and thiols. A direct linkage may also be formed by using an activating agent, such as a carbodiimide, to bind, for example, the primary amino group present on one moiety to the carboxy group present on the other moiety. Activating agents suitable for use in the preparation are well known in the art.

The fucoidan moiety and detectable moiety may be indirectly covalently linked to each other *via* a linker group. This can be accomplished by using any number of stable bifunctional agents well known in the art, including homofunctional and heterofunctional linkers. The use of a bifunctional linker differs from the use of an activating agent in that the former results in a linking moiety being present in the inventive imaging agent after reaction, whereas the latter results in a direct coupling between the two moieties involved in the reaction. The main role of the bifunctional linker is to allow the reaction between two otherwise chemically inert moieties. However, the bifunctional linker, which becomes part of the reaction product, can also be selected such that it confers some degree of conformational flexibility to the imaging agent (e.g., the bifunctional linker may comprise a straight alkyl chain containing several atoms).

A wide range of suitable homofunctinal and heterofunctional linkers known in the art can be used in the context of the preparation. Preferred linkers include, but are not limited to, alkyl and aryl groups, including straight chain and branched alkyl groups, substituted alkyl and aryl groups, heteroalkyl and heteroaryl groups, that have reactive chemical functionalities such as amino, anhydride, hydroxyl, carboxyl, carbonyl groups, and the like.

Methods of direct or indirect covalent association may be used, for example, in the synthesis of selectin-targeted imaging agents comprising a fluorescent moiety. Similarly, such methods may be employed for the coating of USPIO particles by fucoidan moieties (see Example 3), or to graft fucoidan onto acoustically active microbubbles or liposomes (see Example 4).

The fucoidan and detectable moieties may be directly but non-covalently associated to each other. Non-covalent associations include, but are not limited to, hydrophobic interactions, electrostatic interactions, dipole interactions, van der Waals interactions, and hydrogen bonding. For example, a fucoidan moiety and a detectable metal entity may be associated by complexation. Suitable complexation methods include, for example, direct incorporation of the metal entity into the fucoidan moiety and transmetallation. When possible, direct incorporation is preferred. In such a method, an aqueous solution of the fucoidan moiety is generally exposed to or mixed with a metal salt. The pH of the reaction mixture may be between about 4 and about 11. Direct incorporation methods are well known in the art and different procedures have been described (see, for example, WO 87/06229). The present Applicants have shown that a low molecular weight fucoidan can easily be complexed to technetium-99m (see Example 2). A method of transmetallation is used when the metal entity needs to be reduced to a different oxidative state before incorporation. Transmetallation methods are well known in the art. It is to be understood that, given the short lifetime of certain radionuclides *(e.g.,* ^{99m}Tc), the direct incorporation may have to be performed shortly prior to the use of the imaging agent.

When direct, non-covalent association between the fucoidan and detectable metal moieties is not possible, the selectin-targeted imaging agent may comprise at least one fucoidan moiety associated with at least one detectable moiety, wherein the detectable moiety comprises a metal-chelating moiety complexed to a detectable metal moiety. The association between the fucoidan moiety and the metal-chelating moiety is preferably covalent. Suitable metal-chelating moieties may be any of a large number of metal chelators and metal complexing molecules known to bind detectable metal moieties. Preferably, metal-chelating moieties are stable, non-toxic entities that bind radionuclides or paramagnetic metal ions with high affinity.

Examples of metal-chelating moieties that have been used for the complexation of paramagnetic metal ions, such as gadolinium III (Gd³⁺), include DTPA (diethylene triaminepentaacetic acid); DOTA (1,4,7,10-tetraazacyclododecane-*N,N',N",N"'-*tetraacetic acid); and derivatives thereof (see, for example, U.S. Pat. Nos. 4,885,363; 5,087,440; 5,155,215; 5,188,816; 5,219,553; 5,262,532; and 5,358,704; and D. Meyer et al., Invest. Radiol. 1990, 25: S53-55), in particular, DTPA-bis(amide) derivatives (U.S. Pat. No. 4,687,659). Other metal-chelating moieties that complex paramagnetic metal ions include acyclic entities such as aminopolycarboxylic acids and phosphorus oxyacid analogues thereof (e.g., triethylenetetraminehexaacetic acid or TTHA), and dipyridoxal diphosphate (DPDP) and macrocyclic entities (e.g., 1,4,7,10-tetraazacyclododecane-N,N',N"-triacetic acid or DO3A). Metal-chelating moieties may also be any of the entities described in U.S. Pat. Nos. 5,410,043; 5,277,895; and 6,150,376; or in F.H. Arnold, Biotechnol. 1991, 9: 151-156.

Examples of metal-chelating moieties that complex radionuclides, such as technetium-99m, include, for example, N₂S₂ and N₃S chelators (A.R. Fritzberg et al., J. Nucl. Med. 1982, 23:592-598; U.S. Pat. Nos. 4,444,690; 4,670,545; 4,673,562; 4,897,255; 4,965,392; 4,980,147; 4,988,496; 5,021,556 and 5,075,099). Other suitable metal-chelating moieties can be selected from polyphosphates (*e.g.*, ethylene diaminetetramethylenetetra-phosphonate, EDTMP); aminocarboxylic acids (*e.g*., EDTA, N-(2-hydroxy)ethylene-diaminetriacetic acid, nitrilotriacetic acid, N,N-di(2-hydroxyethyl)glycine, ethylenebis(hydroxyphenylglycine) and diethylenetriamine pentacetic acid); 1,3-diketones (*e.g.,* acetylacetone, trifluoroacetylacetone, and thenoyltrifluoroacetone); hydroxycarboxylic acids (*e.g.*, tartaric acid, citric acid, gluconic acid, and 5-sulfosalicyclic acid); polyamines (*e.g.*, ethylenediamine, diethylenetriamine, triethylenetetraamine, and triaminotriethylamine); aminoalcohols (*e.g.*, triethanolamine and N-(2-hydroxyethyl)ethylenediamine); aromatic heterocyclic bases (*e.g.,* 2,2'-diimidazole, picoline amine, dipicoline amine and 1,10-phenanthroline); phenols (*e.g.*, salicylaldehyde, disulfopyrocatechol, and chromotropic acid); aminophenols (*e.g.*, 8-hydroxyquinoline and oximesulfonic acid); oximes (*e.g.*, hexamethylpropyleneamine oxime, HMPAO); Schiff bases (*e.g.*, disalicylaldehyde 1,2-propylenediimine); tetrapyrroles (*e.g.*, tetraphenylporphin and phthalocyanine); sulfur compounds (*e.g*., toluenedithiol, meso-2,3-dimercaptosuccinic acid, dimercaptopropanol, thioglycolic acid, potassium ethyl xanthate, sodium diethyldithiocarbamate, dithizone, diethyl dithiophosphoric acid, and thiourea); synthetic macrocyclic compounds (*e.g.*, dibenzo[18]crown-6), or combinations of two or more of the above agents.

As can readily be appreciated by those skilled in the art, a selectin-targeted imaging agent as described herein can comprise any number of fucoidan moieties and any number of detectable moieties, linked to one another by any number of different ways. The fucoidan moieties within an imaging agent may be all identical or different. Similarly, the detectable moieties within an inventive imaging agent may be all identical or different. The precise design of a selectin-targeted imaging agent will be influenced by its intended purpose(s) and the properties that are desirable in the particular context of its use

### II - Uses of Selectin-Targeted Imaging Agents

The invention provides reagents and strategies to image and detect the presence of selectins. More specifically, the invention provides targeted reagents that are detectable by imaging techniques and methods that allow the detection, localization and/or quantification of selectins in *in vitro* and *ex vivo* systems as well as in living subjects, including human patients. The methods described herein are based on the use of selectin-targeted imaging agents comprising at least one fucoidan moiety having a high affinity and specificity for selectins, associated with at least one detectable moiety that allows visualization of the imaging agent using imaging techniques.

More specifically, the present document describes methods for detecting the presence of selectins in a biological system comprising the step of contacting the biological system with an effective amount of a selectin-targeted imaging agent, or a pharmaceutical composition thereof. The contacting is preferably carried out under conditions that allow the imaging agent to interact with selectins present in the system so that the interaction results in the binding of the imaging agent to the selectins. The imaging agent that is bound to selectins present in the system is then detected using an imaging technique. One or more images of at least part of the biological system may be generated.

The contacting may be carried out by any suitable method known in the art. For example, the contacting may be carried out by incubation.

The biological system may be any biological entity that can produce and/or contain selectins. For example, the biological system may be a cell, a biological fluid or a biological tissue. The biological system may originate from a living subject (e.g., it may be obtained by drawing blood, or by biopsy) or a deceased subject (e.g., it may be obtained at autopsy). The subject may be human or another mammal. The biological system may originate from a patient suspected of having a clinical condition associated with selectins.

The present document also describes methods for detecting the presence of selectins in a patient. The methods comprise administering to the patient an effective amount of a selectin-targeted imaging agent, or a pharmaceutical composition thereof. The administration is preferably carried out under conditions that allow the imaging agent (1) to reach the area(s) of the patient's body that may contain abnormal selectins (*i.e.,* selectins associated with a clinical condition) and (2) to interact with such selectins so that the interaction results in the binding of the imaging agent to the selectins. After administration of the selectin-targeted imaging agent and after sufficient time has elapsed for the interaction to take place, the imaging agent bound to abnormal selectins present in the patient is detected by an imaging technique. One or more images of at least part of the body of the patient may be generated.

Administration of the selectin-targeted imaging agent, or pharmaceutical composition thereof, can be carried out by any suitable method known in the art such as administration by oral and parenteral methods, including intravenous, intraarterial, intrathecal, intradermal and intracavitory administrations, and enteral methods.

As mentioned above, the imaging agent bound to selectins (present either in a biological system or in a patient) is detected using an imaging technique such as contrast-enhanced ultrasonography, planar scintigraphy, SPECT, fluorescence spectroscopy, or a combination thereof.

The methods described herein that provide for detecting the presence of selectins in a patient or in a biological system obtained from a patient can be used to diagnose a pathological condition associated with selectins. The diagnosis can be achieved by examining and imaging parts of or the whole body of the patient or by examining and imaging a biological system (such as one or more samples of biological fluid or biological tissue) obtained from the patient. One or the other method, or a combination of both, will be selected depending of the clinical condition suspected to affect the patient. Comparison of the results obtained from the patient with data from studies of clinically healthy individuals will allow determination and confirmation of the diagnosis.

These methods can also be used to follow the progression of a pathological condition associated with selectins. For example, this can be achieved by repeating the method over a period of time in order to establish a time course for the presence, localization, distribution, and quantification of "abnormal" selectins in a patient.

These methods can also be used to monitor the response of a patient to a treatment for a pathological condition associated with selectins. For example, an image of part of the patient's body that contains "abnormal" selectins (or an image of part of a biological system originating from the patient and containing "abnormal" selectins) is generated before and after submitting the patient to a treatment. Comparison of the "before" and "after" images allows the response of the patient to that particular treatment to be monitored.

Pathological conditions that may be diagnosed, or whose progression can be followed by the methods described herein may be any disease and disorder known to be associated with selectins, *i.e*., any condition that is characterized by undesirable or abnormal interactions mediated by selectins. Examples of such conditions that may advantageously be diagnosed using methods described herein include, but are not limited, thrombosis, myocardial ischemia/reperfusion injury, stroke and ischemic brain trauma, neurodegenerative disorders, tumor metastasis and tumor growth, and rheumatoid arthritis.

### III - Pharmaceutical Compositions

In the methods of detection/imaging of selectins and of diagnosis of pathological conditions associated with selectins described herein, the imaging agents of the present invention may be used *per se* or as a pharmaceutical composition. Accordingly, in one aspect, the present document describes the use of fucoidan for the manufacture of a composition for the diagnosis of clinical conditions associated with selectins. In another aspect, the present document describes pharmaceutical compositions comprising at least one selectin-targeted imaging agent (or any physiologically tolerable salt thereof), and at least one pharmaceutically acceptable carrier.

The specific formulation will depend upon the selected route of administration. Depending on the particular type of pathological condition suspected to affect the patient and the body site to be examined, the imaging agent may be administered locally or systemically, delivered orally (as solids, solutions or suspensions) or by injection (for example, intravenously, intraarterially, intrathecally (*i.e., via* the spinal fluid), intradermally or intracavitory).

Often, pharmaceutical compositions will be administered by injection. For administration by injection, pharmaceutical compositions of imaging agents may be formulated as sterile aqueous or non-aqueous solutions or alternatively as sterile powders for the extemporaneous preparation of sterile injectable solutions. Such pharmaceutical compositions should be stable under the conditions of manufacture and storage, and must be preserved against the contaminating action of microorganisms such as bacteria and fungi.

Pharmaceutically acceptable carriers for administration by injection are solvents or dispersion media such as aqueous solutions (*e.g.*, Hank's solution, alcoholic/aqueous solutions, or saline solutions), and non-aqueous carriers (*e.g.*, propylene glycol, polyethylene glycol, vegetable oil and injectable organic esters such as ethyl oleate). Injectable pharmaceutical compositions may also contain parenteral vehicles (such as sodium chloride and Ringer's dextrose), and/or intravenous vehicles (such as fluid and nutrient replenishers); as well as other conventional, pharmaceutically acceptable, non-toxic excipients and additives including salts, buffers, and preservatives such as antibacterial and antifungal agents (*e.g.*, parabens, chlorobutanol, phenol, sorbic acid, thirmerosal, and the like). Prolonged absorption of the injectable compositions can be brought about by adding agents that can delay absorption (*e.g.*, aluminum monostearate and gelatin). The pH and concentration of the various components can readily be determined by those skilled in the art.

Sterile injectable solutions are prepared by incorporating the active compound(s) and other ingredients in the required amount of an appropriate solvent, and then by sterilizing the resulting mixture, for example, by filtration or irradiation. The methods of manufacture of sterile powders for the preparation of sterile injectable solutions include vacuum drying and freeze-drying techniques.

In general, the dosage of a selectin-targeted imaging agent (or pharmaceutical composition thereof) will vary depending on considerations such as age, sex and weight of the patient, as well as the particular pathological condition suspected to affect the patient, the extent of the disease, the area(s) of the body to be examined, and the sensitivity of the detectable moiety. Factors such as contraindications, therapies, and other variables are also to be taken into account to adjust the dosage of imaging agent to be administered. This, however, can be readily achieved by a trained physician.

In general, a suitable daily dose of a selectin-targeted imaging agent (or pharmaceutical composition thereof) corresponds to the lowest amount of imaging agent (or pharmaceutical composition) that is sufficient to allow detection/imaging of any relevant *(i.e.,* generally overexpressed) selectin present in the patient. To minimize this dose, it is preferred that administration be intravenous, intramuscular, intraperitoneal or subcutaneous, and preferably proximal to the site to be examined. For example, intravenous administration is appropriate for imaging the cardio/neurovascular system; while intraspinal administration is better suited for imaging of the brain and central nervous system.

### IV - Kits

In another aspect, the present invention describes kits comprising materials useful for carrying out the diagnostic methods of the invention. The diagnostic procedures described herein may be performed by clinical laboratories, experimental laboratories, or practitioners.

A kit comprises may comprise at least one fucoidan and at least one detectable entity, and, optionally, instructions for associating the fucoidan and detectable entity to form a selectin-targeted imaging agent described herein.

The detectable entity is preferably a short-lived radionuclide such as technetium-99m (^{99m}Tc), gallium-67 (⁶⁷Ga), yttrium-91 (⁹¹Y), indium-111 (¹¹¹In), rhenium-186 (¹⁸⁶Re), and thallium-201 (²⁰¹T1). Preferably, the fucoidan and detectable entity are present, in the kit, in amounts that are sufficient to prepare a quantity of imaging agent that is suitable for the detection of selectins and diagnosis of a particular clinical condition in a subject.

In addition, the kit may further comprise one or more of: labeling buffer and/or reagent; purification buffer, reagent and/or means; injection medium and/or reagents. Protocols for using these buffers, reagents and means for performing different steps of the preparation procedure and/or administration may be included in the kit.

The different components included in an inventive kit may be supplied in a solid (*e.g.*, lyophilized) or liquid form. The kits described herein may optionally comprise different containers (*e.g.*, vial, ampoule, test tube, flask or bottle) for each individual component. Each component will generally be suitable as aliquoted in its respective container or provided in a concentrated form. Other containers suitable for conducting certain steps of the preparation methods may also be provided. The individual containers of the kit are preferably maintained in close confinement for commercial sale.

In certain embodiments, a kit further comprises instructions for using its components for the diagnosis of clinical conditions associated with selectins according to a method described herein. Instructions for using the kit according to a method described herein may comprise instructions for preparing an imaging agent from the fucoidan and detectable entity, instructions concerning dosage and mode of administration of the imaging agent obtained, instructions for performing the detection of selectins, and/or instructions for interpreting the results obtained. A kit may also contain a notice in the form prescribed by a governmental agency regulating the manufacture, use or sale of pharmaceuticals or biological products.

### Examples

The following examples describe some of the preferred modes of making and practicing the present invention. However, it should be understood that the examples are for illustrative purposes only Furthermore, unless the description in an Example is presented in the past tense, the text like the rest of the specification, is not intended to suggest that experiments were actually performed or data were actually obtained.

Some of the results reported below were presented in a scientific article (L. Bachelet et al., "Affinity of low molecular weight fucoidan for P-selectin triggers its binding to activated human platelets", Biochim. Biophys. Acta, 2009, 1790: 141-146). Other results reported below were presented at the International Carbohydrate Symposium, Oslo, Norway, July 24^{th}-August 1^{st}, 2008 (L. Bachelet et al., "Fucoidan: A sulfated polysaccharide to target activated platelets in atherosclerosis").

### Example 1: LMW Fucoidans are Highly Specific Ligands of P-Selectin Materials and Methods

**Chemical Products.** Fluorescein isothiocyanate (FITC) was purchased from Fluka (Saint-Quentin Fallavier, France); streptavidin-peroxidase conjugate from Dako (Trappes, France); diaminopropane, sodium cyanobromohydride and peroxidase substrate ABTS from Sigma-Aldrich (Saint-Quentin Fallavier, France); sinapinic acid solution from Bio-Rad Laboratories (Hercules, CA, USA); and the amine coupling kit and running buffer from BIAcore (Uppsala, Sweden).

**Polysaccharides.** The low molecular weight fucoidan (based on sulfated repeating fucose unit; M = 7200 g/M; SO₄ = 30% (w/w)) was prepared from brown seaweed as previously described (A. Nardella et al., Carbohydr. Res., 1996, 289: 201-208). The low molecular weight heparin (M = 5700 g/M; SO₄ = 45% (w/w)) and low molecular weight dextran sulfate (M = 8000 g/M; SO₄ = 52% (w/w)), were supplied from Sigma-Aldrich; and the biotinylated polyacrylamide-type glycoconjugate containing 20% mol SLe^{x} was obtained from Lectinity Holding (Moscow, Russia).

**Biological Compounds.** Recombinant human P-selectin (121-124 kDa by SDS-PAGE) and recombinant human P-selectin/Fc chimera (146-160 kDa by SDS-PAGE) were obtained from R&D Systems (Lille, France); bovine serum albumin (BSA), thrombin receptor-activating peptide (TRAP), and adenosine diphosphate (ADP) from Sigma-Aldrich, and purified peptide and protein standards from Bio-Rad Laboratories.

**Antibodies.** The PC5-labeled IgG (MOPC-21 clone), PC5-labeled antihuman P-selectin (CD62P, AK-4 clone), FITC-labeled IgM and FITC-labeled PAC-1 (directed to active conformation of integrin complex GPIIb/IIIa) were supplied from BD Biosciences (Le Pont de Claix, France); FITC-labeled IgG (MOPC-21 clone), FITC-labeled anti-human CD41 (integrin GPIIb) and goat anti-human Fc IgGperoxidase from Beckman-Coulter (Roissy, France); goat anti-human Fc IgG from Sigma-Aldrich; anti-human P-selectin (CD62P, G1 clone) from COGER (Paris, France).

**Other Materials.** Immulon 1B microtiter plates were a gift from VWR (Fontenay sous Bois, France). Anionic protein chips CM10 were obtained from Bio-Rad Laboratories; CM5 sensor chips from BIAcore.

**FITC Labeling of Polysaccharides.** Five hundred (500) milligrams of polysaccharide and 250 mg of NaBH₃CN were added to 4 mL of diaminopropane hydrochloride solution at 2.5 M. After 24 hours at 60°C, 250 mg of NaBH₃CN were added to the mixture and the reaction was carried on for 48 hours. Samples were dialyzed (cut-off 1000 Da) before freezedrying. One hundred and fifty (150) milligrams of aminated polysaccharide was dissolved in 6 mL of 0.5 M carbonate buffer (pH 9.6). Six (6) milligrams of FITC was added to the solution which was stirred at 4°C in darkness for 2 hours. After neutralization, the solution was dialyzed (cut-off 1000 Da) and freeze-dried. The colored compound was then dissolved at 150 mg/mL in NaCl 1 M, precipitated by ethanol and centrifuged at 4500 rpm for 20 minutes to remove the free fluorescein. Fucoidan was successfully fluorolabeled using this protocol with a grafting of 0.19±0.06 fluorophore per polysaccharide chain.

**P-Selectin Binding Assay with Sialyl Lewis X.** This protocol was adapted from a previously described method (Weitz-Schmidt et al., Anal. Biochem., 1999, 273: 81-88). P-selectin/Fc chimera (5 µg/mL in phosphate buffered saline, PBS, 137 mM NaCl, 8.1 mM Na₂HPO₄ 1.4 mM KH₂PO₄ and 2.7 mM KCl, pH=7.2) was coated onto microtiter plates overnight at 4°C. The plates were washed with the assay buffer (20 mM Hepes, pH 7.4, containing 150 mM NaCl and 1 mM CaCl₂), blocked for 4 hours at 4°C with 3% BSA in the same buffer, and washed again. Polysaccharides or anti-human P-selectin (G1 clone) and biotinylated SLe^{x}-polymer were diluted in the assay buffer and added to the P-selectin-coated wells or the BSA-coated wells (non specific control) for incubation overnight at 4°C. The plates were then washed and streptavidin-peroxidase diluted 1:1000 in the assay buffer was added to the wells. After 4 hours at 4°C, the plates were washed with assay buffer. ABTS peroxidase substrate solution was added and the color reaction was stopped after 10 minutes with 2% oxalic acid. Bound SLe^{x}-polymer was determined by measuring the optical density at 405 nm using a microplate reader.

**P-Selectin Binding Assay with PSGL-1.** P-selectin (5 µg/mL in PBS, 137 mM NaCl, 8.1 mM Na₂HPO₄ 1.4 mM KH₂PO₄ and 2.7 mM KCl, pH=7.2) was coated onto microtiter plates overnight at 4°C. The plates were washed with the assay buffer (20 mM Hepes, pH 7.4, containing 150 mM NaCl and 1 mM CaCl₂), blocked for 4 hours at 4°C with 3% BSA in the same buffer, and washed again. Fucoidan and PSGL-1/Fc chimera were diluted in the assay buffer and added to the P-selectin-coated wells or the BSA-coated wells (non specific control) for incubation overnight at 4°C. The plates were then washed and IgG antiFc-peroxidase diluted 1:1000 in the assay buffer was added to the wells. After 4 hours at 4°C, the plates were washed with the assay buffer. ABTS peroxidase substrate solution was added and the color reaction was stopped after 5 minutes with 2% oxalic acid. Bound PSGL-1 was determined by measuring the optical density at 405 nm using a microplate reader.

**SELDI-TOF Analysis.** Anionic protein chip arrays CM10 were employed. Spots were prewetted twice for 5 minutes with 5 µL of Hepes pH 7.0. Samples were prepared by mixing 500 ng of recombinant human P-selectin in the absence or in the presence of different concentrations of polysaccharides (molar ratio between P-selectin and polysaccharide of 1 per 1 to 1 per 100), diluted in 1 M Hepes pH 7.0, in a total volume of 5 µL, and incubated for 1 hour at 4°C. The samples were applied to the spots and incubated for 45 minutes at room temperature in a humid chamber. The spots were washed three times with 5 µL of 1 M Hepes pH 7.0 and twice with 5 µL of distilled water and then air-dried for 10 minutes. One (1) µL of a saturated solution of sinapinic acid (in 50% acetonitrile, 0.5% trifluoroacetic acid) was applied twice to each spot. The protein chip arrays were analyzed using a protein chip reader (PBS II, Bio-Rad). The protein masses were calibrated externally using purified peptide and protein standards. Spectra were analyzed with protein chip software 3.1.1 (Bio-Rad).

**Surface Plasmon Resonance.** BIAcore 2000 optical biosensor was used. The carboxymethylated dextran surface CM5 sensor chip was coupled with goat anti-human Fc IgG using standard amine coupling chemistry (averaged 6500 RU). Recombinant human P-selectin/Fc chimera was then captured to the chip (averaged 1750 RU). Goat anti-human Fc IgGwas used as non specific control. Samples were diluted in running buffer (10 mM Hepes, 150 mM NaCl, 1 mM CaCl₂, and 0.005% Tween-20, pH 7.4). Flow cell, temperature, flowrate, sample volume, and mixing were selected using the BIAcore control software. Sensorgrams were analyzed using the BIAevaluation software.

**Flow Cytometry.** Blood from healthy adult donors was collected into sodium citrate 3.8% (w/v). Citrated human whole blood (5 µL) was diluted to 40 µL with PBS (137 mM NaCl, 8.1 mM Na₂HPO₄ 1.4 mM KH₂PO₄ and 2.7 mM KCl, pH=7.2). Platelets were activated by adding ADP at a final concentration of 2.5 µM or TRAP at a final concentration of 200 µM. Five µL of LMW polysaccharides (FITC-labeled or not, diluted in PBS) and 5 µL of fluorolabeled antibody (PC5-labeled antiCD62P AK-4 clone or FITC-labeled PAC-1 or FITC-labeled antiCD41; diluted 8:100; 5:100 and 3:100 in PBS) were added at room temperature for 20 minutes. The solutions were diluted to 1 mL with PBS before analysis by flow cytometry. Data were collected on a Coulter EPICS XL-MCL flow cytometer (Beckman Coulter). Samples analysis was performed on side and forward scatter, and fluorescence was acquired in FL1 (fluorescein) or FL4 (PC5) using the logarithmic mode. 7500 events were collected from each sample. The level of platelet activation was assessed by the positivity of the anti-Pselectin (CD62P, AK-4 clone) antibody (0.4%, 73.4%, and 97.8% for non-activated, ADP-activated and TRAP-activated platelets, respectively). Data were processed using GEN S® System II software (Beckman Coulter) and histograms are presented overlapped for the different conditions.

**Statistical Analysis.** Data shown are representative results of at least three identical and independent experiments carried out each time with n≥3 samples per conditions. Statistical comparisons were performed with the Student's t-test.

### Results

**LMW fucoidan inhibits the binding of SLe^{x} and PSGL-1 to P-selectin.** The binding of SLe^{x}-polyacrylamide-biotin to immobilized P-selectin was measured in the presence of LMW fucoidan, heparin and dextran sulfate. In this assay, an anti-human P-selectin antibody (clone G1, as a positive control) completely blocked the binding of SLe^{x} to P-selectin. The amount of SLe^{x} bound to P-selectin decreased with increasing concentrations of polysaccharides. However, major differences were observed between the sulfated polysaccharides (Figure 1). Inhibition by fucoidan was much more pronounced than with heparin and dextran sulfate, with an IC₅₀ of 20 nM, 400 nM and >25,000 nM, respectively. The binding of PSGL-1/Fc chimera to immobilized P-selectin was also evaluated in the presence of LMW fucoidan. The amount of PSGL-1 bound to P-selectin decreased with increasing concentration of fucoidan with an IC₅₀ of 5 nM.

**Binding of LMW fucoidan to P-selectin.** The binding of P-selectin was then analyzed by mass spectrometry and by surface plasmon resonance (SPR).

The formation of a complex between P-selectin and the three LMW sulfated polysaccharides and native dextran was analyzed using SELDI-TOF MS. Anionic chips on which P-selectin bound (isoelelectric point ~6.5) at physiological pH=7 were used. P-selectin was then desorbed by laser and detected as a SELDI-TOF broad peak of ∼100 kDa. The amount of P-selectin markedly decreased in the presence of LMW fucoidan, and in a dose dependent manner. P-selectin retention to the chip also decreased with heparin but was not affected by incubation with native dextran or dextran sulfate. These results demonstrate that LMW fucoidan forms a complex with P-selectin in solution thus preventing its retention to the anionic surface.

The binding characteristics of LMW sulfated polysaccharides to P-selectin were further compared using surface plasmon resonance analysis. LMW fucoidan, heparin and dextran sulfate were flowed on a sensorchip coated either with anti-human Fc IgG or with recombinant human P-selectin/Fc chimera (Figure 2). All polysaccharides bound to P-selectin and, to a lesser extent, to anti-human Fc IgG used as a control. The signal difference obtained on P-selectin *vs* IgG was higher with fucoidan than with heparin or dextran sulfate, which suggests that fucoidan exhibits a better selectivity (Figure 2A). Dissociation constants of LMW fucoidan, heparin and dextran sulfate for P-selectin, calculated using a 1:1 Langmuir binding model (Figure 2D), were found to be 1.2 nM, 577 nM and 118 nM, respectively. These results confirmed that LMW fucoidan has an affinity for P-selectin at least two orders of magnitude higher than the two other polysaccharides.

**Binding of LMW Fucoidan to Human Platelets.** Heparin was previously reported to bind to platelets (J. Hirsh et al., Chest, 2004, 126: 188S-203S; R. Verhaege, Acta Cardiol., 1998, 53: 15-21). Flow cytometry experiments were performed by incubating human citrated whole blood with FITC-labeled LMW fucoidan. A representative experiment is reported on Figure 3. Platelets were gated on side and forward scatter and their positivity for a fluorolabeled specific platelet antibody (CD41). FITC-labeled fucoidan bound to activated platelets as demonstrated by a shift of the fluorescence to the right. Fucoidan binding increased with the level of platelet activation as indicated by the percentage of positive platelets, 34.7%, 51.4%, and 69.1% for nonactivated, ADP-activated and TRAP-activated platelets, respectively.

TRAP-activated platelets were then incubated with a fluorolabeled anti CD62P antibody in whole blood in the presence or in the absence of non-fluorolabeled LMW fucoidan. Inhibition of the CD62P antibody binding to activated platelets was observed in the presence of LMW fucoidan as indicated by a decrease in the mean fluorescence intensity (Figure 4). In addition, LMW fucoidan did not inhibit the binding of CD41 (integrin GPIIb) antibody or PAC-1 (directed to the active conformation of the integrin complex GPIIb/IIIa) to activated platelets, indicating that its effect on CD62P antibody binding to activated platelets was specific. All together, these results indicate that the binding of LMW fucoidan to activated platelets observed in whole human blood was mediated by P-selectin.

### Discussion

Sulfated carbohydrates are known to have a wide variety of biological activities (S. Soeda et al., Biochim. Biophys. Acta, 2000, 1497: 127-137). Sulfated polysaccharides have previously been described as P-selectin ligands (A. Varki et al., PNAS, 1994, 91: 7390-7397; D. Simonis et al., Biochemistry, 2007, 46: 6156-6164) *e.g.,* heparin and modified heparins (A. Koenig et al., J. Clin. Invest., 1998, 101: 877-889), high molecular weight fucoidan and dextran sulphate (M.P. Skinner et al., J. Biol. Chem., 1991, 266: 5371-5374). In the present study, the interaction of three low molecular weight sulfated polysaccharides (fucoidan, heparin and dextran sulfate) with P-selectin was characterized using four different methods. LMW fucoidan is a promising candidate for the treatment of inflammation disorders (K. Senni et al., Arch. Biochem. Biophys., 2006, 445: 56-64) and cardiovascular diseases (33; 34; F. Zemani et al., Arterioscler. Thromb. Vasc. Biol., 2008, 28: 644-650). LMW heparin is used in the treatment of thrombotic disorders (K.A. Fox et al., Eur. Heart J., 2000, 21: 1440-1449). Synthetic dextran sulfate and mimetics were also investigated as putative drugs in various diseases, including infection diseases (J. Neyts et al., Biochem. Pharmacol., 1995, 50: 743-751).

The inhibition of SLe^{x}/P-selectin binding was quantified in binding assay experiments ranking polysaccharides as follows (IC₅₀): fucoidan (20 nM) > heparin (400 nM) > dextran sulfate (25,000 nM). As a comparison, Koenig *et al.* established by inhibition assays that heparin inhibited P-selectin binding to Sialyl Lewis X with IC₅₀ between 82 and 2400 µM depending on the size of the heparin fragment (A. Koenig et al., J. Clin. Invest., 1998, 101: 877-889). However, in their work, Sialyl Lewis X was immobilized whereas, in the present approach, it is P-selectin that was immobilized. The functional importance of LMW fucoidan binding to P-selectin was evidenced by the interference in the interaction between the glycoprotein with its natural ligand PSGL-1.

SELDI-TOF mass spectrometry was used to highlight the formation of a complex between P-selectin and LMW polysaccharides. This tool has allowed to demonstrate the binding of heparin and fucoidan to thrombin and protease nexin-I (B. Richard et al., Thromb. Haemost., 2006, 95: 229-235). SELDI-TOF MS experiments showed that, in solution, LMW fucoidan formed a complex with P-selectin at physiological pH in a dose-dependent manner. The complex formation decreased P-selectin retention to an anionic surface.

The interaction of sulfated polysaccharides such as heparin or fucoidan with various proteins has been previously studied by surface plasmon resonance (BIAcore®) (32; H. Yu et al., Biochim. Biophys. Acta, 2005, 1726: 168-176). For instance, it was shown that a SLe^{x} mimetic binds to P-selectin with K_{D} of 114 µM (M.E. Beauharnois et al., Biochemistry, 2005, 44: 9507-9519) and PSGL-1 binds to P-selectin with K_{D} of 320 nM (P. Mehta et al., J. Biol. Chem., 1998, 273: 32506-32513). The dissociation constant of LMW heparin for P-selectin calculated here, with a K_{D} above 500 nM, is in the same range as those of three unfractioned heparins determined by quartz crystal microbalance measurements in the study of Simonis et al. (Biochemistry, 2007, 46: 6156-6164). Interestingly, this results demonstrated that LMW fucoidan with a K_{D} in the nanomolar range is the most effective and selective P-selectin ligand when compared with other LMW polysaccharides, PSGL-1 and the SLe^{x} mimetic. Moreover, P-selectin/LMW fucoidan interaction is stronger than the L-selectin/ GlyCAM-1 interaction, also involved in leukocyte rolling on blood vessels endothelium. The interaction constant of this interaction was determined to be 108 µM by Nicholson et al. (J. Biol. Chem., 1998, 273: 763-770).

Native and fractionated heparins were shown to interact with P-selectin on HL-60 cells (Y. Gao et al., Mol. Cells, 2005, 19: 350-355). In order to determine whether the binding of fucoidan to P-selectin observed using purified proteins could occur in more complex conditions, the interaction of LMW polysaccharides with human platelets on whole blood was analyzed. Using flow cytometry, LMW fucoidan was found to bind to activated platelets and the level of binding was found to correlate with the degree of platelet activation. Moreover, LMW fucoidan was able to inhibit the binding of an anti P-selectin antibody to activated human platelets.

### Example 2: ^{99m}Tc-labelled Fucoidan as P-selectin-targeted Imaging Agent for the in vivo Scintigraphic Detection of Platelet Activation and Accumulation

Fucoidan was labelled with technetium-99m (^{99m}Tc) using the classical stannous reaction in solution. Briefly, 4 µL of stannous chloride followed by 2 µL of potassium borohydride were added to 10 µL of fucoidan (1 mg/mL, MW=7200). Immediately after combination of these reagents, 50 µL of ^{99m}Tc (corresponding to 15-30 mCi) were gently added to the mixture. The labelling reaction was complete after 1 hour of incubation. Control of the labelling was performed using thin layer paper chromatography and methyl-ketone as eluant. The percentage of labelling was 100%.

Rat models of endocarditic vegetations, aneurysmal and atrial trombi were used as animal models of clinical conditions associated with platelet activation and fibrin formation. Intravenous injection of 1 µg of ^{99m}Tc-labelled fucoidan allowed the *in vivo* visualization of platelet-rich endocarditic vegetations (Figure 5), atrial (Figure 6) and aneurismal thrombi (Figure 7). These *in vivo* data were confirmed by *ex vivo* autoradiography showing the exact histological co-localization of the signal with valves vegetations or thrombus with a very high quantitative signal to background ratio of 8 to 10.

The development of fucoidan as a radiotracer for selectin imaging can be considered at several steps: (1) ability to visualize P-selectin overexpression by acutely-activated endothelium (ischemia-reperfusion model); (2) ability to visualize E-selectin overexpression by chronically-stimulated endothelium (L-NAME model of hypertension); and (3) ability or not to visualize L-selectin accumulation in tertiary lymph node formation (aortic allograft in rats) and in auto-immune myocarditis.

### Example 3: Preparation of fucoidan-coated USPIO Particles

### (not part of the claimed invention)

30 The present Applicants have developed five different strategies to coat fucoidan onto USPIO particles.

The first strategy involves the synthesis of iron particles in the presence of unmodified fucoidan. The Applicants have applied a method of synthesis previously described with dextran (R.S. Molday et al., J. Immunol. Methods, 1982, 52: 353-367) replacing dextran MW 40000 by fucoidan MW 50500. Fucoidan-coated iron nanoparticles were obtained. However, these particles were found to be unstable in water.

The second strategy comprises the coating of an acidic ferrofluid with unmodified fucoidan. Fucoidan was incubated with acidic ferrofluid. Fucoidan-coated iron nanoparticles were obtained that were stable in aqueous medium pH 7.4, but unstable in buffers with ionic strength of 0.15 M, which are used in most applications. The synthesis could be obtained in the presence of a cross-linker (A. San Juan et al., J. Biomed. Mater Res. A, 2007, 82: 354-362) to increase the stability of the nanoparticles.

Three other strategies have been developed that are based on the grafting of fucoidan to maghemite γFe₂O₃ nanoparticles *via* a linker (strategy 3), or the coupling of fucoidan to dextan-coated maghemite γFe₂O₃ nanoparticles, *i.e.,* coated with carboxymethyl dextran or CMD (strategy 4) or coated with oxidised dextran (strategy 5). In all these strategies, the first step is to functionalize the reducing end of fucoidan with a primary amine to allow subsequent reaction without altering the fucoidan chain structure and therefore its affinity for P-selectin.

In strategy 3, the iron nanoparticles were thiolated with dimercaptosuccinic acid (DMSA) as previously described (French patent No. 2 736 197; N. Fauconnier et al., J. Colloid Interface Sci., 1997, 194: 427-433), and then linked to the aminated fucoidan by a disulfide bridge using a heterobifunctional linker, N-succinimidyl 3-(2-pyridyldithio) propionate (SPDP) (J. Roger et al., Eur. Phys. J. Applied Phys., 1999, 5: 321-325). Iron nanoparticles coated with a LMW fucoidan (MW = 7200) were obtained that were found to be stable in usual aqueous buffer pH 7.4 with ionic strength of 0.15 M.

In strategy 4, CMD (MW = 15000) was incubated with acidic ferrofluid to obtain CMD-coated iron nanoparticles stable in usual aqueous buffer pH 7.4 with ionic strength of 0.15 M. Aminated fucoidan is grafted on these nanoparticles using standard amine coupling chemistry (with EDC/NHS system).

In strategy 5, aminated fucoidan is grafted to oxidized dextran-coated nanoparticles by formation of Schiff bases.

### Example 4: Preparation of Fucoidan-coated Acoustically Active Microbubbles and Liposomes

### (not part of the claimed invention)

Several strategies are investigated to graft fucoidan on ultrasound-based imaging contrast agents. In a first approach, aminated fucoidan is grafted to phospholipids-based, perfluorobutane-filled microbubbles using standard amine coupling chemistry as previously described by Villanueva et al. (Circulation, 1998, 98: 1-5). Briefly, perfluorobutane was dispersed by sonication in aqueous medium containing phosphatidylcholine, a surfactant, a phosphatidylethanolamine derivative and a phospholipid derivative containing carboxyl groups which were activated with 1-ethyl-3(3-dimethylaminopropyl) carbodiimide (EDC) and aminated fucoidan was then covalently attached *via* primary amino groups with the formation of amine bonds.

A second strategy is to graft biotinylated fucoidan to phospholipid-based, perfluorobutane-filled microbubbles *via* a multi-step avidin/biotin bridging chemistry as previously described by Weller et al. (Biotechnol. Bioeng., 2005, 92: 780-788). Briefly, an aqueous saline solution containing phosphatidylcholine, polyethylene glycol stearate and a biotinylated derivative of phosphatidylethanolamine was sonicated with perfluorobutane. The microbubbles formed were incubated with streptavidin, and then a saturating amount of biotinylated fucoidan.

Biotinylated fucoidan was obtained using a method previously described by Osmond et al. (Anal. Biochem., 202, 310(2), 199-207) for the biotinylation of an aminated heparin. Briefly, sulfosuccinimidyl-6-(biotinamido)hexanoate-(sulfo-NHS-LC-biotin) was added to a solution of aminated fucoidan in carbonate buffer 0.1 M pH=8 with a molar ratio between aminated fucoidan and sulfo-NHS-LC-biotin of 1 to 10. The mixture was vortexed and shaken overnight at 4°C, and was then dialyzed (cut-off 1000 Da) against bi-distilled water before freeze-drying.

Finally, a third approach is to graft fucoidan to acoustically active liposomes using a thiol chemistry as previously described by H.amilton et al. (Circulation, 2002, 105: 2772-2778). Briefly, component phospholipids (phosphatidylcholine, phosphatidylglycerol, phosphatidylethanolamine derivative and cholesterol) were dissolved in chloroform and mixed and the resulting film was sonicated in water to form liposomes; aminated fucoidan was reacted with 3-(2-pyridylthio)propionic acid-N-hydroxysuccinimide ester (SPDP). The fucoidan derivative was then reduced in dithiothreitol solution and the thiolated fucoidan was then conjugated to liposomes.

### Example 5: Fucoidan Substituted with lodinated Tyrosine for X-ray Radiography

### (not part of the claimed invention)

Another possibility for the radio labelling of fudoidan is the use of sodium iodide after grafting of a tyrosine residue onto the reductive end of the polysaccharide. The substitution step is similar to the amination step described in Example 1.

Briefly, 200 mg of polysaccharide and 100 mg of NaBH₃CN were added to 1.6 mL of a tyrosine hydrochloride solution at 2.5 M. After 24 hours at 60°C, 100 mg of NaBH₃CN were added to the mixture and the reaction was prolonged for 48 hours. The sample was dialyzed (cut-off 1000 Da) against bidistilled water before freeze-drying; Modified fucoidan was recovered in a 50-70% yield with a grafting of 0.05 of tyrosine per polysaccharide chain.

Iodination was performed using chloramine-T as follows: 20 µmoles of modified fucoidan (14.5 mg) in 450 µL of phosphate buffer saline 0.05 M, pH 7.4 (PBS) were added to a NaI solution (150 µL of 8% w/v solution in PBS) followed by the addition of 350 µL of a chloramine-T solution (40 mg/mL in PBS). The mixture was vortexed, and shaken overnight at 4°C. The reaction mixture was then dialyzed against bidistilled water (cut off 1000 Da) and freeze-dried to get the iodinated modified fucoidan in quantitative yield.

### Other Embodiments

Other embodiments of the invention will be apparent to those skilled in the art from a consideration of the specification or practice of the invention disclosed herein. It is intended that the specification and examples be considered as exemplary only, with the true scope of the invention being indicated by the following claims.

## Claims

1. An imaging agent comprising at least one fucoidan directly complexed to at least one detectable moiety, wherein the imaging agent is selectin-targeted, wherein the detectable moiety is detectable by planar scintigraphy (PS) or Single Photon Emission Computed Tomography (SPECT), and wherein the detectable moiety is selected from the group consisting of technetium-99m (^{99m}Tc), gallium-67 (⁶⁷Ga), yttrium-91 (⁹¹Y), indium-111 (¹¹¹In), rhenium-186 (¹⁸⁶Re), and thallium-201 (²⁰¹T1).

2. The selectin-targeted imaging agent according to claim 1, wherein the at least one fucoidan binds at least one human selectin selected from the group consisting of P-selectin, L-selectin and E-selectin with a dissociation constant of between about 0.1 nM and about 500 nM, preferably between about 0.5 nM and about 10 nM, more preferably between about 1 nM and about 5 nM.

3. The imaging agent according to claim 1 or claim 2, wherein the at least one fucoidan has an average molecular weight of about 2000 to about 8000 Da, or of about 20,000 to about 70,000 Da, or of about 100,000 to about 500,000 Da.

4. A pharmaceutical composition comprising an effective amount of at least one selectin-targeted imaging agent according to any one of claims 1-3, or a physiologically tolerable salt thereof, and at least one pharmaceutically acceptable carrier.

5. A selectin-targeted imaging agent according to any one of claims 1-3 for use in the detection and/or imaging of selectins.

6. A selectin-targeted imaging agent according to any one of claims 1-3 or a pharmaceutical composition according to claim 4 for use in the diagnosis of a clinical condition associated with selectins.

7. A selectin-targeted imaging agent or a pharmaceutical composition for the use according to claim 6, wherein the clinical condition associated with selectins is a member of the group consisting of thrombosis, myocardial ischemia/reperfusion injury, stroke and ischemic brain trauma, neurodegenerative disorders, tumor metastasis and tumor growth, and rheumatoid arthritis.

8. A method for detecting the presence of abnormal selectins in a biological sample, the method comprising steps of:
contacting the biological sample with an effective amount of a selectin-targeted imaging agent according to any one of claims 1-3 or a pharmaceutical composition according to claim 4, and
detecting any selectin bound to the imaging agent using an imaging technique.

9. The method according to claim 8, wherein the biological sample is selected from the group consisting of a cell, a biological fluid and a biological tissue.

10. The method according to claim 8, wherein the biological sample is a biological sample obtained from a patient suspected of having a clinical condition associated with selectins, and said method is used to diagnose the clinical condition associated with selectins or wherein the biological system is a biological system obtained a patient who has received a treatment for a clinical condition associated with selectins, and said method is used to monitor the response of the patient to the treatment.

11. The method according to claim 10, wherein the clinical condition associated with selectins is a member of the group consisting of thrombosis, myocardial ischemia/reperfusion injury, stroke and ischemic brain trauma, neurodegenerative disorders, tumor metastasis and tumor growth, and rheumatoid arthritis.

12. A kit for the *in vitro* diagnosis of a clinical condition associated with selectins in a patient or for the detection of abnormal selectins in a biological system, the kit comprising a fucoidan, a detectable moiety selected from the group consisting of technetium-99m (^{99m}Tc), gallium-67 (⁶⁷Ga), yttrium-91 (⁹¹Y), indium-111 (¹¹¹In), rhenium- 186(¹⁸⁶Re), and thallium-201 (²⁰¹T1), and instructions for preparing a selectin-targeted imaging agent according to any one of claims 1-3; and optionally further comprising instructions for diagnosing the clinical condition using the selectin-targeted imaging agent and/or instructions for detecting abnormal selectins in the biological system using the selectin-targeted imaging agent.

## Patentansprüche

1. Ein Bildgebungsagens, umfassend mindestens Fucoidan, das direkt an mindestens eine detektierbare Gruppe komplexiert ist, wobei das Bildgebungsagens Selectin-gerichtet ist, wobei die detektierbare Gruppe durch planare Szintigraphie (PS) oder Einzelphoton-Emissions-Computer-Tomographie (SPECT) detektierbar ist, und wobei die detektierbare Gruppe ausgewählt ist aus der Gruppe, bestehend aus Technetium-99m (^{99m}Tc), Gallium-67 (⁶⁷Ga), Yttrium-91 (⁹¹Y), Indium-111 (¹¹¹In), Rhenium-186 (¹⁸⁶Re), und Thallium-201 (²⁰¹TI).

2. Das Selectin-gerichtete Bildgebungsagens nach Anspruch 1, wobei das mindestens eine Fucoidan mindestens ein humanes Selectin bindet, ausgewählt aus der Gruppe, bestehend aus P-Selectin, L-Selectin and E-Selectin mit einer Dissoziationskonstante von zwischen ungefähr 0.1 nM und ungefähr 500 nM, vorzugsweise zwischen ungefähr 0.5 nM und ungefähr 10 nM, stärker bevorzugt zwischen ungefähr 1 nM und ungefähr 5 nM.

3. Das Bildgebungsagens nach Anspruch 1 oder Anspruch 2, wobei das mindestens eine Fucoidan ein mittleres Molekulargewicht von ungefähr 2000 bis ungefähr 8000 Da, oder von ungefähr 20,000 bis ungefähr 70,000 Da, oder von ungefähr 100,000 bis ungefähr 500,000 Da hat.

4. Eine pharmazeutische Zusammensetzung, umfassend eine wirksame Menge von mindestens einem Selectin-gerichteten Bildgebungsagens nach einem der Ansprüche 1 bis 3, oder ein physiologisch tolerierbares Salz davon, und mindestens einen pharmazeutische akzeptablen Träger.

5. Ein Selectin-gerichtetes Bildgebungsagens nach einem der Ansprüche 1 bis 3 zur Verwendung bei der Detektion und/oder Bildgebung von Selectinen.

6. Ein Selectin-gerichtetes Bildgebungsagens nach einem der Ansprüche 1 bis 3 oder eine pharmazeutische Zusammensetzung nach Anspruch 4 zur Verwendung in der Diagnose eines klinischen Zustands, der mit Selectinen assoziiert ist.

7. Ein Selectin-gerichtetes Bildgebungsagens oder eine pharmazeutische Zusammensetzung zur Verwendung nach Anspruch 6, wobei der klinische Zustand, der mit Selectinen assoziiert ist, ein Mitglied der Gruppe, bestehend aus Thrombose, myokardialer Ischämie/Reperfusions-Schaden, Schlaganfall und ischämischem Gehirntrauma, neurogenerativen Erkrankungen, Tumor-Metastasierung und Tumorwachstum, und Rheumatoider Arthritis, ist.

8. Ein Verfahren zur Bestimmung der Präsenz von abnormalen Selectinen in einer biologischen Probe, wobei das Verfahren die Schritte umfasst des:
Kontaktierens der biologischen Probe mit einer effektiven Menge eines Selectingerichteten Bildgebungsagents nach einem der Ansprüche 1 bis 3 oder einer pharmazeutischen Zusammensetzung nach Anspruch 4, und
Detektierens irgendeines Selectins, das an das Bildgebunsagens gebunden ist, unter Verwendung einer Bildgebungstechnik.

9. Das Verfahren nach Anspruch 8, wobei die biologische Probe ausgewählt ist aus der Gruppe, bestehend aus einer Zelle, einer biologischen Flüssigkeit und einem biologischen Gewebe.

10. Das Verfahren nach Anspruch 8, wobei die biologische Probe eine biologische Probe ist, die aus einem Patienten erhalten wird, der Verdacht auf einen klinischen Zustand hat, der mit Selectinen assoziiert ist, und wobei das Verfahren verwendet wird, um den klinischen Zustand, der mit Selectinen assoziiert ist, zu diagnostizieren, und wobei das biologische System ein biologisches System, ist, das aus einem Patienten erhalten wird, der eine Behandlung des klinischen Zustands, assoziiert mit Selectinen, erhalten hat, und wobei das Verfahren verwendet wird, um die Antwort des Patienten auf die Behandlung nachzuverfolgen.

11. Das Verfahren nach Anspruch 10, wobei der klinische Zustand, der mit Selectinen assoziiert ist, ein Mitglied der Gruppe, bestehend aus Thrombose, myokardialer Ischämie/Reperfusions-Schaden, Schlaganfall und ischämischem Gehirntrauma, neurogenerativen Erkrankungen, Tumor-Metastasierung und Tumorwachstum, und Rheumatoider Arthritis, ist.

12. Ein Kit zur *in vitro* Diagnose eines klinischen Zustands, der mit Selectinen assoziiert ist, in einem Patienten oder zur Detektion von abnormalen Selectinen in einem biologischen System, wobei der Kit ein Fucoidan umfasst, eine detektierbare Gruppe, ausgewählt aus der Gruppe bestehend, aus Technetium-99m (^{99m}Tc), Gallium-67 (⁶⁷Ga), Yttrium-91 (⁹¹Y), Indium-111 (¹¹¹In), Rhenium-186 (¹⁸⁶Re), und Thallium-201 (²⁰¹TI), und Instruktion zur Herstellung eines Selectin-gerichteten Bildgebungsagens nach einem der Ansprüche 1 bis 3; und optional weiter umfassend Instruktionen zur Diagnose des klinischen Zustands unter Verwendung des Selectin-gerichteten Bildgebungsagens und/oder Instruktion zur Detektion von abnormalen Selectinen in dem biologischen System unter Verwendung des Selectin-gerichteten Bildgebungsagens.

## Revendications

1. Agent d'imagerie comprenant au moins un fucoïdane directement complexé à au moins un groupement détectable, dans lequel l'agent d'imagerie cible une sélectine, dans lequel le groupement détectable est détectable par une scintigraphie planaire (PS) ou par une tomographie d'émission monophotonique assistée par ordinateur (SPECT), et dans lequel le groupement détectable est choisi dans le groupe constitué du technétium-99m (^{99m}Tc), du gallium-67 (⁶⁷Ga), de l'yttrium-91 (⁹¹Y), de l'indium-111 (¹¹¹In), du rhénium-186 (¹⁸⁶Re) et du thallium-201 (²⁰¹T1).

2. Agent d'imagerie ciblant une sélectine selon la revendication 1, dans lequel le au moins un fucoïdane se lie à au moins une sélectine humaine choisie dans le groupe constitué de la sélectine P, de la sélectine L et de la sélectine E avec une constante de dissociation comprise entre environ 0,1 nM et environ 500 nM, de préférence entre environ 0,5 nM et environ 10 nM, de manière davantage préférée entre environ 1 nM et environ 5 nM.

3. Agent d'imagerie selon la revendication 1 ou la revendication 2, dans lequel le au moins un fucoïdane a une masse moléculaire moyenne d'environ 200 à environ 8 000 Da, ou d'environ 20 000 à environ 70 000 Da ou d'environ 100 000 à environ 500 000 Da.

4. Composition pharmaceutique comprenant une quantité efficace d'au moins un agent d'imagerie ciblant une sélectine selon l'une quelconque des revendications 1 à 3, ou d'un sel physiologiquement tolérable de celui-ci, et au moins un véhicule pharmaceutiquement acceptable.

5. Agent d'imagerie ciblant une sélectine selon l'une quelconque des revendications 1 à 3 pour une utilisation dans la détection et/ou l'imagerie de sélectines.

6. Agent d'imagerie ciblant une sélectine selon l'une quelconque des revendications 1 à 3 ou composition pharmaceutique selon la revendication 4 pour une utilisation dans le diagnostic d'une pathologie clinique associée à des sélectines.

7. Agent d'imagerie ciblant une sélectine ou composition pharmaceutique pour une utilisation selon la revendication 6, dans lequel la pathologie clinique associée à des sélectines est un membre du groupe constitué de la thrombose, d'une ischémie du myocarde/lésion de reperfusion, une attaque et un traumatisme ischémique cérébral, des troubles neurodégénératifs, d'une métastase de tumeur et d'une croissance de tumeur et de la polyarthrite rhumatoïde.

8. Procédé de détection de la présence de sélectines anormales dans un échantillon biologique, le procédé comprenant les étapes de :
mise en contact de l'échantillon biologique avec une quantité efficace d'un agent d'imagerie ciblant une sélectine selon l'une quelconque des revendications 1 à 3 ou une composition pharmaceutique selon la revendication 4, et
détection de toute sélectine liée à l'agent d'imagerie par une technique d'imagerie.

9. Procédé selon la revendication 8, dans lequel l'échantillon biologique est choisi dans le groupe constitué d'une cellule, d'un fluide biologique et d'un tissu biologique.

10. Procédé selon la revendication 8, dans lequel l'échantillon biologique est un échantillon biologique obtenu auprès d'un patient suspecté de présenter une pathologie clinique associée à des sélectines, et ledit procédé est utilisé pour diagnostiquer la pathologie clinique associée à des sélectines ou dans lequel le système biologique est un système biologique obtenu auprès d'un patient qui a reçu un traitement pour une pathologie clinique associée à des sélectines, et ledit procédé est utilisé pour suivre la réponse du patient au traitement.

11. Procédé selon la revendication 10, dans lequel la pathologie clinique associée à des sélectines est un membre du groupe constitué de la thrombose, d'une ischémie du myocarde/lésion de reperfusion, une attaque et un traumatisme ischémique cérébral, des troubles neurodégénératifs, d'une métastase de tumeur et d'une croissance de tumeur et de la polyarthrite rhumatoïde.

12. Kit pour le diagnostic *in vitro* d'une pathologie clinique associée à des sélectines chez un patient ou pour la détection de sélectines anormales dans un système biologique, le kit comprenant un fucoïdane, un groupement détectable choisi dans le groupe constitué du technétium-99m (^{99m}Tc), du gallium-67 (⁶⁷Ga), de l'yttrium-91 (⁹¹Y), de l'indium-111 (¹¹¹In), du rhénium-186 (¹⁸⁶Re) et du thallium-201 (²⁰¹T1), et des instructions de préparation d'un agent d'imagerie ciblant une sélectine selon l'une quelconque des revendications 1 à 3 ; et éventuellement comprenant en outre des instructions pour le diagnostic de la pathologie clinique à l'aide de l'agent d'imagerie ciblant une sélectine et/ou des instructions de détection de sélectines anormales dans le système biologique à l'aide de l'agent d'imagerie ciblant une sélectine.
